# EUROPEAN PATENT SPECIFICATION

(11) **EP 3 426 766 B1**
(45) Date of publication and mention of the grant of the patent: **28.12.2022**
(21) Application number: 17714913.5
(22) Date of filing: 08.03.2017
(51) Int. Cl.: C12M 3/06, C12M 1/00, C12M 1/34, G01N 33/50

(54) **METHOD FOR CONTINUOUS BIOSENSING**
VERFAHREN ZUM KONTINUIERLICHEN BIOSENSING
PROCÉDÉ DE BIODÉTECTION CONTINUE

(30) Priority: 08.03.2016 US 201662305073 P; 09.03.2016 US 201662305660 P
(43) Date of publication of application: 16.01.2019
(73) Proprietor: Yissum Research and Development Company of the Hebrew University of Jerusalem Ltd., 9139002 Jerusalem (IL)
(72) Inventor: NAHMIAS, Yaakov, 7570239 Rishon-LeZion (IL); LEVY, Gahl, 5258241 Ramat-Gan (IL); BAVLI, Danny, 9987500 Tzur-Hadassa (IL)
(74) Representative: Patentanwälte Bauer Vorberg Kayser
(86) International application number: PCT/IL2017/050289
(87) International publication number: WO 2017/153992

(56) References cited:
- WO-A1-2013/086505
- US-A1- 2015 268 224
- DANNY BAVLI ET AL: "Real-time monitoring of metabolic function in liver-on-chip microdevices tracks the dynamics of mitochondrial dysfunction", PROCEEDINGS NATIONAL ACADEMY OF SCIENCES PNAS, vol. 113, no. 16, 19 April 2016 (2016-04-19), pages E2231-E2240, XP055375339, US ISSN: 0027-8424, DOI: 10.1073/pnas.1522556113
- PRILL SEBASTIAN ET AL: "Real-time monitoring of oxygen uptake in hepatic bioreactor shows CYP450-independent mitochondrial toxicity of acetaminophen and amiodarone", ARCHIVES OF TOXICOLOGY, SPRINGER, DE, vol. 90, no. 5, 4 June 2015 (2015-06-04), pages 1181-1191, XP035866592, ISSN: 0340-5761, DOI: 10.1007/S00204-015-1537-2 [retrieved on 2015-06-04]
- EZRA ELISHAI ET AL: "Microprocessor-based integration of microfluidic control for the implementation of automated sensor monitoring and multithreaded optimization algorithms", BIOMEDICAL MICRODEVICES, KLUWER, DORDRECHT, NL, vol. 17, no. 4, 31 July 2015 (2015-07-31), pages 1-9, XP035527019, ISSN: 1387-2176, DOI: 10.1007/S10544-015-9989-Y [retrieved on 2015-07-31]

## Description

The present invention relates to a method of biosensing of cells.

Prescription drugs and cosmetics are used for extensive periods of time, and their development requires demonstration of long-term safety. Major causes of damage include hepatotoxicity, cardiotoxicity, and neurotoxicity, and nephrotoxicity.

Microfluidic liver-on-chip devices offer an alternative to animal experiments as they can mimic the native microenvironment and support long-term function under continuous perfusion. One advantage of microfluidics is the ability to expose cells to a stable stimulation over time, eliminating the rapid loss of signal due to non-specific adsorption and metabolism that characterizes both static *in vitro* assays and *in vivo.* Stable microfluidic stimulation permits the acquisition of reliable information about the effect of a specific dose, rather than the response of cells to a rapidly changing drug concentration.

U.S. Patent No. 9,170,253 discloses an apparatus for determining a cell constituent. The apparatus has moveable barriers that reduce a volume of media in contact with samples in a multiwell plate, and sensors disposed on respective surfaces of the barriers in sensing contact with the media in the wells. The barriers and sensors are inserted into respective wells of the multiwell plate. When the barriers and sensors are moved into the wells, the volume of the media about the cells in the wells is reduced, thus defining a reduced-volume sample chamber in contact with the sample. The constituents of the cells are analyzed using the sensors within the reduced volume. Thereafter, the barriers are moved to retain the original volume.

Additional background art includes U.S. Patent Nos. 9,170,255 and 8,202,702, and U.S. Published Application No. 20140170671, Prill et al., 2015 Arch Toxicol, 10.1007/s00204-015-1537-2, US 2015/268224 A1 and WO 2013/086505 A1.

Therefore, it is an object of the present invention to provide a method for biosensing of cells that one the one hand compensates for the drawbacks of the state of the art and on the other hand provides an alternative of methods. This object is solved by a method according to claim 1.

According to the present invention there is provided a method for biosensing cells, comprising:
(a) subjecting the cells to a continuous single-pass perfusion wherein said perfusion comprises a perfusion medium which comprises a proliferation inhibitor, wherein said cells comprise microparticles or nanoparticles embedded therebetween, wherein said microparticles or nanoparticles serve as oxygen sensors,
(b) measuring glucose and lactate directly using electrochemical sensors, wherein electrical signal from said electrochemical sensors and optical signals from said oxygen sensors are collected over the same time period, and
(c) analyzing signals of said oxygen sensors and of said electrochemical sensors to thereby determine one or more physiological parameters that are characteristic to the cells.

According to an embodiment of the present invention, said one or more physiological parameters is glycolytic flux of the cells.

According to a further embodiment of the present invention, said one or more physiological parameters indicative of said glycolytic flux is glycolytic capacity.

According to a further embodiment of the present invention, said glycolytic capacity is determined following perfusion with a saturating concentration of glucose.

According to a further embodiment of the present invention, said glycolytic flux is glycolytic reserve.

According to a further embodiment of the present invention, said glycolytic reserve is determined following perfusion with a glycolysis inhibitor.

According to a further embodiment of the present invention, said glycolysis inhibitor is 2-deoxyglucose, which inhibits glycolysis by binding to hexokinase.

According to a further embodiment of the present invention, said glycolytic flux is non-glycolytic acidification.

According to a further embodiment of the present invention, said one or more physiological parameters is mitochondrial function.

According to a further embodiment of the present invention, said mitochondrial function is basal respiration.

According to a further embodiment of the present invention, said mitochondrial function is ATP-linked respiration, H⁺ leak, maximal respiration or spare respiratory capacity.

According to a further embodiment of the present invention, said one or more physiological parameters is non-mitochondrial respiration.

According to a further embodiment of the present invention, said non-mitochondrial respiration is determined following perfusion of rotenone and antimycin A.

According to a further embodiment of the present invention, said perfusion medium comprises a glycolysis inhibitor, a mitochondrial pyruvate carrier (MPC) inhibitor, a glutaminase GLS1 inhibitor and/or a Carnitine Palmitoyltransferase 1A (CPT1A) inhibitor.

According to a further embodiment of the present invention, said perfusion medium comprises glucose.

According to a further embodiment of the present invention, said perfusion medium comprises at least one of a drug, a drug candidate, a toxin and a nutrient and wherein the method comprising assessing change of oxygen uptake, change of glucose uptake and change of lactate production in the presence of the at least one of a drug, a drug candidate, a toxin and a nutrient.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method of the invention can involve performing or completing selected tasks manually, automatically, or a combination thereof. Moreover, according to actual instrumentation and equipment of embodiments of the method of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data.

Optionally, a network connection is provided as well. A display and/or a user input device such as a keyboard or mouse are optionally provided as well.

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIGs. 1A-B are schematic illustrations of a Bioanalyzer.
FIG. 2A is a schematic of glucose and glutamine utilization by central carbon metabolism.
FIG. 2B is a schematic illustration of a measurement setup.
FIG. 2C is a Jablonski diagram describing the generation of phosphorescence with Ru-CPOx beads under the influence of oxygen.
FIG. 2D demonstrates that the effect described in FIG. 2C induces a phase shift between the intensity-modulated excitation and emission light.
FIG. 2E schematically illustrates a design of amperometric sensor in 2-electrode configuration.
FIG. 2F is a schematic illustration describing that that H₂O₂ is created in equivalent amounts of the analyte as an intermediate product by the activity of glucose oxidase (GOx) or lactate oxidase (LOx).
FIG. 3A is a schematic explosive view of a bioreactor.
FIG. 3B is an image of assembled bioreactor on microscope stage.
FIGs. 3C1 and 3C2 show HepG2/C3A organoid after overnight incubation with embedded oxygensensing micro-probes (orange).
FIGs. 3D1 and 3D2 show results of numerical simulation of the pressure drop and variations in oxygen concentration throughout the bioreactor.
FIGs. 3E1 and 3E2 show cross-section of oxygen gradient developing due to consumption within the well (from top to bottom), mimicking the *in vivo* microenvironment.
FIG. 3F shows representative long term oxygen measurement over 1 month in bioreactor perfused with cell culture medium at 2 µl/min.
FIG. 3G shows log-scale quantitative gene expression analysis of HepG2/C3A cells in static culture compared to those perfused with 1% DMSO for 30 days (growth arrest).
FIG. 4A shows representative oxygen uptake over time response of HepG2/C3A cells exposed to increasing concentrations of rotenone.
FIG. 4B shows dose dependence of rotenone after 12 hours. TC50 was calculated to be 12.5 µM.
FIG. 4C demonstrates that TUNEL^{™} staining shows 14-fold increase in apoptosis following 24 hours exposure to 200 µM rotenone (p<0.001, n=5), as well as unlabeled cell death suggesting necrosis.
FIG. 4D shows representative oxygen uptake over time response of HepG2/C3A cells exposed to increasing concentrations of troglitazone.
FIG. 4E shows dose dependence of troglitazone after 24 hours. TC50 was calculated to be 285 µM.
FIG. 4F demonstrates that CDFDA staining shows 4.5-fold increase in intracellular accumulation of bile acids (cholestasis) following 24 hours exposure to 200 µM troglitazone (p<0.001, n=5).
FIG. 4G is a IVIVC curve comparing TC50 values of primary human hepatocytes to the bioreactor showing an excellent R2=0.99 correlation for acetaminophen, amiodarone, troglitazone, and rotenone.
FIG. 4H demonstrates that the time to onset of mitochondrial damage was dose-dependent for both rotenone (left) and troglitazone (right), occurring in minutes rather than hours following exposure to the drugs.
FIG. 4I shows oxygen consumption rate (OCR) measured on isolated mitochondria for 30 min, followed by ADP injection (arrow) and subsequent injection of 50 µM rotenone (left) or troglitazone (right).
FIG. 5A is an image of PMMA unit housing both glucose and lactate sensors with total internal volume of 26 µL.
FIGs. 5B1 and 5B2 show amperometric calibration curves of glucose (FIG. 5B1) and lactate (FIG. 5B2) sensors in the PMMA housing.
FIG. 5C is an image of two-layer microfluidic switchboard containing flow channels (red) and independently addressed control channels (blue).
FIG. 5D shows characterization of micromechanical valve switching pressure as a function of number of cycles (age).
FIG. 5E is a schematic illustration of microfluidic switchboard connectivity and operating sequence.
FIG. 5F shows images of perfusion sequence starting with a washing step (blue), air purging, and sample introduction (red).
FIG. 5G is an image of a simplified set-up where a single bioreactor is connected to the switchboard.
FIG. 5H shows automatic amperometric calibration and measurement of glucose concentration in bioreactor outflow.
FIG. 6A shows Live/Dead staining of HepG2/C3A organoids in bioreactors treated for 24 hours with 50 or 200 µM rotenone.
FIG. 6B shows representative curves of oxygen uptake, glucose uptake, and lactate production of HepG2/C3A cells following exposure to 50 µM rotenone (dotted line).
FIG. 6C shows changes in lactate over glucose ratio following exposure to rotenone (dotted line).
FIG. 6D shows Live/Dead staining of HepG2/C3A organoids in bioreactors treated for 24 hours with 50 or 200 µM troglitazone.
FIG. 6E shows representative curves of oxygen uptake, glucose uptake, and lactate production of HepG2/C3A cells following exposure to 50 µM troglitazone (dotted line).
FIG. 6F shows changes in lactate over glucose ratio following exposure to troglitazone (dotted line).
FIG. 6G shows relative ATP production rate calculated from flux balance analysis, juxtaposed with experimentally measured ATP/ADP ratio in troglitazone, rotenone, and vehicle treated cells.
FIG. 6H shows diverging metabolic sources of ATP production in untreated cells (normal), troglitazone-treated cells (mitochondrial stress) and rotenone-treated cells (mitochondrial dysfunction) presented as pie charts of relative diameter.
FIG. 7A shows live staining of HepG2/C3A cells with JC1 dye following exposure to 50 µM rotenone (top) or troglitazone (bottom).
FIG. 7B demonstrates that rotenone causes significant increase in mitochondrial membrane potential (MMP) after 3 hours of exposure.
FIG. 7C shows direct measurement of oxygen consumption rate (OCR) using a Seahorse XF24 analyzer, on HepG2/C3A cells exposed to 50 µM troglitazone, rotenone, or vehicle control.
FIG. 7D demonstrates that troglitazone induced 20±6% decrease in basal metabolic rate (p<0.01, n=3) and a 22±9% decrease in maximal mitochondrial capacity (p<0.05, n=3) following CCCP injection.
FIG. 7E shows that extracellular acidification rate (ECAR), a surrogate measure of lactate production, increased by 43±43% in troglitazone-treated cells, and decreased 21±12% in rotenone-treated cells (p>0.17, n=3). ^{∗} p<0.05, ^{∗∗} p<0.001 by student's T-test.
FIGs. 8A-B are schematic illustrations depicting the metabolic response of liver cells to troglitazone-induced mitochondrial stress and rotenone-induced mitochondrial dysfunction compared to untreated controls.
FIG. 9A shows images of HepG2/C3A cells stained for PAS and hematoxylin, following 12 hours exposure to drugs.
FIG. 9B demonstrates that glycogen content is unchanged between three conditions.
FIG. 10A shows glutamine uptake (outlet-inlet) in HepG2/C3A bioreactors exposed to 50 µM troglitazone or rotenone (dotted arrow).
FIG. 10B shows glutamine uptake rate, averaged over 3-6 hours, during exposure to troglitazone, rotenone, or control.
FIGs. 11A-C illustrate how deficiency of particular nutrients in the perfusion medium eliminates particular pathways of the cells.

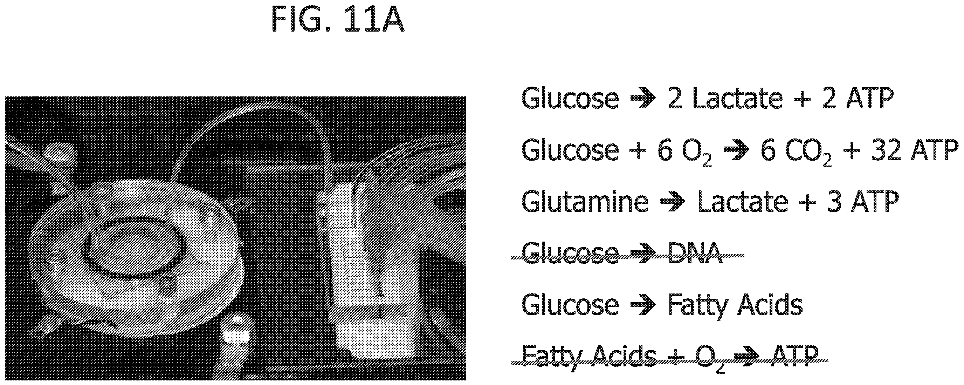

FIG. 11A illustrates that when fatty acids are limited in the medium, lipid oxidation can be neglected for the sake of measuring metabolic flux. When cells do not proliferate, the pentose phosphate pathway (PPP) pathway can be neglected for the sake of measuring metabolic flux.

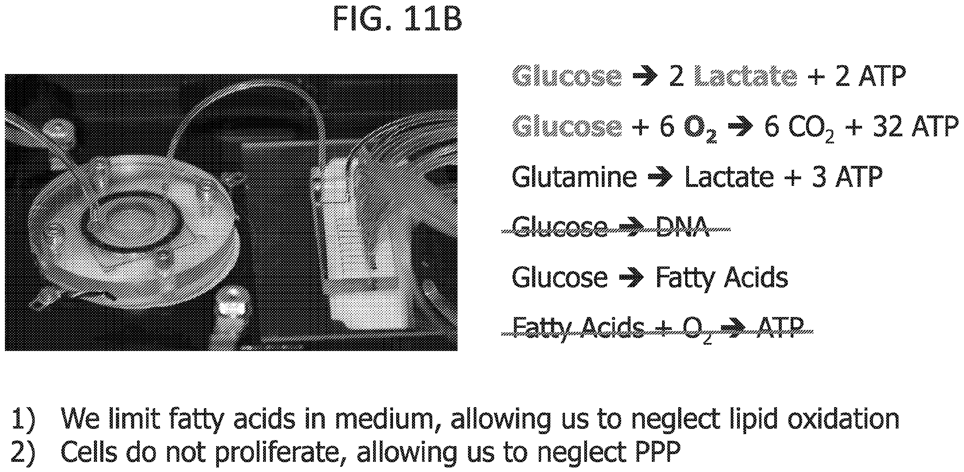

FIG. 11B illustrates that when fatty acids are limited in the medium, lipid oxidation can be neglected for the sake of measuring metabolic flux. When cells do not proliferate, the PPP pathway can be neglected for the sake of measuring metabolic flux. Measurement of Lactate production and glucose uptake allow glycolysis to be calculated. Measurement of glucose and oxygen uptake, allow oxidative phosphorylation to be calculated.

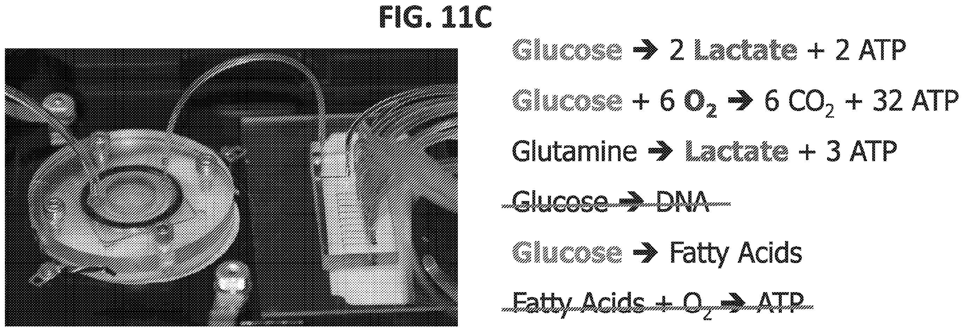

FIG. 11C illustrates that when fatty acids are limited in the medium, lipid oxidation can be neglected for the sake of measuring metabolic flux. When cells do not proliferate, the PPP pathway can be neglected for the sake of measuring metabolic flux. Measurement of Lactate production and glucose uptake allow glycolysis to be calculated. Measurement of glucose and oxygen uptake, allow oxidative phosphorylation to be calculated. Excess lactate must come from glutamine, allowing for the calculation of glutaminolysis. Excess glucose goes to fatty acids allowing for the calculation of lipogenesis.

The present invention relates to a method for biosensing of cells comprising the features given in claim 1.

Except when explicitly stated, the following description may include examples, aspects, embodiments which are not directed to a method according to the invention as claimed in claim 1. However, features not being explicitly referred to as features "according to the invention" may be preferred (optional) features of the invention.

Mitochondrial dysfunction plays a critical role in the development of chemical and pharmaceutical toxicity, as well as pluripotency and disease processes. However, current methods to evaluate mitochondrial activity still rely on end-point assays, resulting in limited kinetic and prognostic information. The present inventors have now devised a microfluidic device capable of maintaining human tissue for over a month in vitro under physiological conditions. Mitochondrial respiration was monitored in real time using two-frequency phase modulation of tissue-embedded phosphorescent microprobes. A computer-controlled microfluidic switchboard allowed contiguous electrochemical measurements of glucose and lactate, providing real-time analysis of minute shifts from oxidative phosphorylation to anaerobic glycolysis, an early indication of mitochondrial stress.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

The present invention provides a method for biosensing of cells. For example, the method of the present embodiments can be used for long-term monitoring of one or more physiological parameters of a cell, including but not limited to mitochondrial respiration.

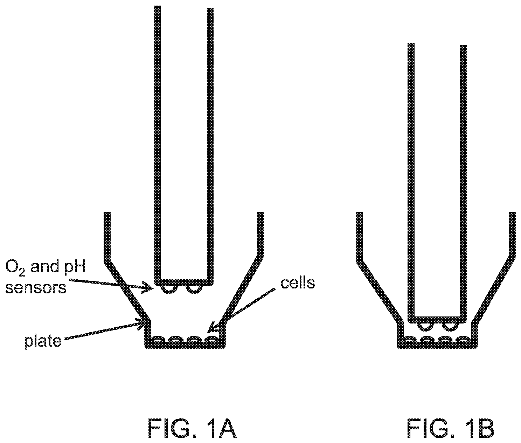

The method according to the present invention employs continuous single-pass perfusion. This is unlike the bioanalyzer described above in connection with FIGs. 1A-B, in which continuous single-pass perfusion is not possible due to the lowering of the sensors between successive measurements.

The term "continuous single-pass perfusion," as used herein, refers to a process in which fresh medium is fed into a measurement chamber at the same rate as spent medium is removed from the chamber, wherein the feeding and removing are generally simultaneous, and wherein perfusion begins before, continues while, and ends after, the sensing of signals from the cells in the chamber.

Preferably, the method of the present invention are carried out in a microfluidics system.

The phrase "single-pass" as used herein, refers to the single direction of the flow (i.e. non-circulating).

The advantage of employing continuous single-pass perfusion is that it allows the cells to be stabilized under steady state conditions without ischemia. Another advantage is that it provides substantially constant (with deviation of less than 10%) drug concentration, since non-specific absorption sites are saturated and the cell metabolism reaches a steady state. This is unlike the bioanalyzer described above in connection with FIGs. 1A-B, in which the drug concentration is decaying.

According to the present invention, the method employs microparticles or nanoparticles embedded between the cells. Preferably, the microprobes or nanoparticles are lifetime-based luminescence-quenching (LBLQ) microparticles or nanoparticles. The microparticles or nanoparticles are optionally and preferably used for measuring oxygen by determining their phase modulation. According to the present invention, the microparticles or nanoparticles serve as oxygen sensors. The advantage of using microparticles or nanoparticles as an oxygen sensor is that such oxygen measurement can be done without calibrating the number of cells and there is no need to operate in tiny volumes.

Microparticles and nanoparticles useful for use as an oxygen sensor suitable for the present embodiments are found in U.S. Published Application No. 20150268224, published on September 24, 2015.

As well as measuring the amount of oxygen in the system outflow (so as to determine oxygen consumption of the cells), the present inventors envisage measuring lactate in the system outflow (so as to determine lactate production of the cells) and/or measuring glucose in the system outflow (so as to determine glucose usage of the cells).

According to the present disclosure, but not being part of the invention as claimed in claim 1, the system further comprises a glutamine sensor.

In some embodiments of the presentAccording to the method of the present invention glucose and lactate are measured directly. This is contrary to the bioanalyzer described above in connection with FIGs. 1A-B which relays on surrogate measures of lactate production, such as acidulation rate and the like.

According to the present disclosure, but not being part of the invention as claimed in claim 1, the oxygen measurement is independent of optical focus, thereby permitting rapid high-throughput screening of hundreds of wells with little delay for focus acquisition and relatively simple robotic setups.

According to the present disclosure, but not being part of the invention as claimed in claim 1, the oxygen sensor is insensitive to amplitude. The advantage of these embodiments is that processes such as cell migration, aggregation, and necrosis have little or no effect on measurement stability.

According to the present disclosure, but not being part of the invention as claimed in claim 1, a plate containing the cells to be analyzed and microparticles or nanoparticles embedded between the cells is placed in an apparatus suitable for biosensing. The apparatus can be of any type suitable for biosensing. For example, the apparatus can be similar to the apparatus described in U.S. Patent No. 9,170,253 *supra.* In these embodiments, the apparatus is preferably operated without bringing the barriers into contact with the plate or medium containing the cells, and without reducing the volume in which the measurement is performed.

A perfusion element is used for generating, in a controlled manner, a flow of a perfusion medium onto the plate, wherein the flow is controlled so as to ensure continuous single-pass perfusion, as defined hereinabove. During the flow, signals indicative of one or more physiological parameters are collected from the plate. The signals can be recorded on a computer readable medium, preferably a non-transitory computer readable medium. Alternatively or additionally, the signals can be analyzed to determine one or more physiological parameters that are characteristic to the cells on the plate.

Suitable media for use in the system of the present embodiments and suitable measuring protocols are found in U.S. Patent No. 9,170,253 *supra.* When these protocols are employed, the barriers are preferably not brought into contact with the plate or medium containing the cells, and the volume in which the measurement is performed is preferably not reduced.

Typically, but not necessarily, the plate has a plurality of microwells, wherein the cells are within the microwells. Alternatively, but also not necessarily, the plate can have one or more microchannels wherein the cells are within the microchannels.

The cells are typically immobilized on a solid surface of the microfluidics device. Any method of immobilization is conceived by the present inventors as long as the cells remain viable.

According to a particular embodiment of the present disclosure, but not being part of the invention as claimed in claim 1" the cells are attached (either directly, or non-directly, e.g. via a gel or other such element) to the outer surface of a solid surface of the microfluidics device (e.g. plate, channel or well).

Optionally and preferably the microchannels are aligned such that the direction of flow of the perfusion medium has a vector component that is orthogonal to the microchannels. According to an embodiment of the present disclosure, but not being part of the invention as claimed in claim 1" each microwell or microchannel has a diameter of 75 to 3000 micrometers. In another embodiment according to the present disclosure, but not being part of the invention as claimed in claim 1" each microwell or microchannel has a height of 25 to 1000 micrometers. According to an embodiment of the present disclosure, but not being part of the invention as claimed in claim 1" the plate is flat-faced. The cells may be introduced by perfusion into a closed plate, or they can be directly seeded on an open plate.

The system according to an embodiment of the present disclosure, but not being part of the invention as claimed in claim 1, can employ more than one type of sensors. According to an embodiment of the present disclosure, but not being part of the invention as claimed in claim 1,the system comprises an oxygen sensor, according to an embodiment of the present disclosure, but not being part of the invention as claimed in claim 1, the system comprises a glucose sensor, and according to an embodiment of the present disclosure, but not being part of the invention as claimed in claim 1,the system comprises a lactate sensor. Any combination of two of these three types of sensors is contemplated, and a system including all three types of sensors is also contemplated.

At least one the three sensors may be independently electrochemically operated. Alternatively each of the sensors may be composed of micro- or nanoparticles typically fluorescent or phosphorescent. According to an embodiment of the present disclosure, but not being part of the invention as claimed in claim 1,the oxygen sensor is made of micro- or nanoparticles and the lactate and/or glucose sensors are electrochemical - e.g. allowing for amperometric measurement of lactate and/or glucose. According to another embodiment of the present disclosure, but not being part of the invention as claimed in claim 1,all three types of sensors are micro- nanoparticles. According to an embodiment of the present disclosure, but not being part of the invention as claimed in claim 1" one or more of the oxygen, lactate and glucose sensors are integral to the system.

According to an embodiment of the present disclosure, but not being part of the invention as claimed in claim 1,pH of the outflow is measured as a surrogate for lactate measurement.

Sensors which are of fluorescent or phosphorescent particles (whether oxygen, lactate or glucose) may be operated according to the principle of a detected change of frequency, phase and/or amplitude of signals (*e*.*g*., optical signals) received from the particles.

The system of the embodiments of the present disclosure, but not being part of the invention as claimed in claim 1, may further comprise at least one of: an electronic control circuit for signal modulation and read-out, a light source *(e.g.,* LED) for excitation *(e.g.,* of the oxygen sensing particles), an optical filter set *(e.g.,* 531/40, 555, 607/70 nm) and a detector unit containing a photomultiplier (PMT). One skilled in the art would appreciate that the sensing particles can be excited by various wave lengths depending on the specific sensing particles used. Accordingly, emission may be read at various wave lengths as well. According to an embodiment of the present disclosure, but not being part of the invention as claimed in claim 1, the oxygen sensing particle is a ruthenium-phenanthroline-based particle, the particles are excited by 532 nm and a 605 nm emission is read, so as to measure phosphorescence decay, substantially in real time.

The system of embodiments of the present disclosure, but not being part of the invention as claimed in claim 1optionally and preferably comprises microfluidic switchboard which is controlled by the electronic control circuit. The microfluidic switchboard preferably comprises a plurality of flow channels, a plurality of micromechanical valves (for example, pressure-controlled valves), and a plurality of independently addressed control channels, for automating washing, calibration and/or collection of electrical signals from the electrochemical sensors (in embodiments in which such sensors are employed). The switchboard can also introduce samples, gas *(e.g.,* air), washing buffer and/or calibration buffer into the electrochemical sensors, in response to control signals received from the electronic circuit according to a predetermined automated protocol.

According to the method of the present invention, the electrical signals from the electrochemical sensors and the optical signals from the particles are collected over the same time period.

In a representative example, which is not to be considered as limiting, the system comprises particles as the oxygen sensor, and electrochemical cells as the glucose and lactate sensors, in which case the electronic control circuit preferably ensures that oxygen uptake rate is monitored optically, and glucose uptake and lactate production are monitored electrically over the same time period.

Glutamine, like glucose and lactate, can also be measured electrochemically. For example, glutaminase and glutamate oxidase enzymes may be immobilized in a membrane. Glutamine is transformed to glutamic acid by glutaminase, and the glutamic acid is transformed by glutamate oxidase to form a detectable reaction product using amperometric or potentiometric sensor. Sensors can be purchased from Innovative Sensor Technologies (Las Vegas, NV). Other methods of measuring glutamine production are described in WO1988010424 A1, US 4,780,191.

Usually, glutaminase and glutamate oxidase enzymes are immobilized in a membrane. Glutamine is transformed to glutamic acid by glutaminase, and the glutamic acid is transformed by glutamate oxidase to form a detectable reaction product using amperometric or potentiometric sensor. Sensors can be purchased from Innovative Sensor Technologies (Las Vegas, NV).

The electronic control circuit optionally and preferably signals the microfluidic switchboard to repeatedly execute a predetermined sequence of operations over a sufficiently long time-period (*e*.*g*., at least a day, or at least a week, or at least two weeks, or at least four weeks). The operations can include at least one operation, more preferably at least two operations, more preferably at least three operations, more preferably each of the operations selected from the group consisting of sampling of outflow from the wells or channels of the plate, washing, recalibrating the electrochemical sensors and air purging. Preferably, air purging is executed before and after (*e.g*., a few seconds before and a few seconds after) any operation in which liquid (*e*.*g*., washing buffer, sample medium, calibration buffer) is delivered to the electrochemical sensors. In a representative example, which is not to be considered as limiting, the following sequence of operations is executed: washing, air purging, outflow sampling, air purging, washing, air purging, calibration, air purging, washing.

While liquid perfusion medium is delivered to the plate, the constituent in the medium may be analyzed to determine any changes, and the measurements can be repeated with or without changing the contents of the delivered medium. Any number of constituents may be analyzed, including, without limitation dissolved gasses, ions, proteins, metabolic substrates, salts, and minerals. These constituents may be consumed by the cells (such as O₂), or may be produced by the cells either as a byproduct (such as CO₂ and NH₃) or as a secreted factor (such as insulin, cytokines, chemokines, hormones, or antibodies). Ions such as H⁺, Na⁺, K⁺, and Ca⁺⁺ secreted or extracted by cells in various cellular metabolism processes may also be analyzed. Substrates either consumed or produced by cells such as glucose, fatty acid, amino acids, glutamine, glycogen, and pyruvate may be analyzed. Specialized media may be used to improve the sensitivity of the measurement. For example, a change in pH resulting from extracellular acidification can be increased by using a media with reduced buffer capacity, such as bicarbonate-free media.

The method of the present embodiments can be used to measure any number of attributes of cells and cellular function. For example, cell viability and metabolic rate may be determined from measurements of oxygen consumption rate, extracellular acidification rate, or other metabolic analyte fluxes. By comparison of one or more analyte flux rates to a known rate per cell, cell number may be determined and therefore growth rates can be monitored.

The present invention contemplates analyzing any cell type including for example bacteria, fungus, yeast, a prokaryotic cell, a eukaryotic cell, an animal cell, a human cell and an immortal cell.

The cells may comprise hepatic cells, neuronal cells, pancreatic cells, muscle cells, skin cells, retinal cells, bone cells, cardiac cells etc.In one embodiment, the cells are derived from an organ including but not limited to the liver, heart, pancreas, brain, skeletal muscle etc.

The cells may be healthy cells (i.e. derived from a healthy subject) or diseased cells (e.g. cancer cells, necrotic cells etc.).

The cells which are analyzed may be comprised in three dimensional aggregates, e.g. of more than about 100 cells - e.g. as spheroids.

The cells may be non-proliferating. In one embodiment, the cells are primary cells. In another embodiment the cells have been contacted with an agent that prevents proliferation - e.g. DMSO. In another embodiment, the perfusion medium is one that prevents proliferation (e.g. Bicarbonate (NaHCO₃)-free Medium). Alternatively, the perfusion medium comprises a proliferation inhibitor such as dichloroacetate, 6-Aminonicotinamide, epiandrosterone, or dehydroepiandrosterone.

Other examples of proliferation inhibitors include but are not limited to pretreatment with mitomycin C, exposure to gamma radiation or serum starvation.

The term "non-proliferating" as used herein refers to the proliferation state of the cells, such that the amount of proliferation or replication thereof is substantially reduced during the time of the measurements. This is to ensure that the amount of glucose oxidation through the pentose phosphate pathway is at least 10, 20, 50 or even 100 times lower than the amount of glucose oxidation through glycolysis.

According to an embodiment of the present disclosure, but not being part of the invention as claimed in claim 1, the amount of glucose oxidation through the pentose phosphate pathway is at least 50 times lower than the amount of glucose oxidation through glycolysis.

According to another embodiment of the present disclosure, but not being part of the invention as claimed in claim 1, the cells do not replicate at all during the course of the measuring.

The present embodiments also contemplate perfusion of media which comprises an environment altering constituent such as a chemical, dissolved gas, nutrient, and a ligand (e.g., a ligand that activates a transmembrane receptor). One or more measurements are optionally and preferably made in the presence of the environment altering constituent. After this measurement cycle, the media may be exchanged one or more times to flush out the environment altering constituent before exposing cells to another medium.

According to an embodiment of the present disclosure, but not being part of the invention as claimed in claim 1, the constituents of the perfusion are such that at least one metabolic pathway of the cells is eliminated. Thus for example the perfusion medium may be deficient in at least one nutrient type.

It will be appreciated that the term "deficient" does not necessarily mean that the medium is totally devoid of that constituent, but that it may be present in limited amounts such that the at least one metabolic pathway of the cells is eliminated. Thus, for example trace amounts of the constituent may be present in the proliferation medium.

Elimination of a pathway (i.e. bypassing of a pathway, or shunting away from the use of the pathway) also does not have to be total. In one embodiment, utilization of that pathway is at least 10 times, 20 times, 50 times or even 100 times lower than an alternate pathway which generates the same end-product and/or which uses the same starting material.

It will be appreciated that the term "elimination of a pathway" may refer to the bypassing of, or shunting away from, only one or both directions of a pathway.

Elimination of a pathway is important such that when metabolic flux is calculated this pathway (or pathway in one particular direction) can be neglected.

The eliminated pathway includes for example the lipid oxidation pathway, glycolysis, glutaminolysis, urea cycle, lipogenesis, cholesterol synthesis, mevalonate pathway (bile acid production), and the anaplerotic reactions replenishing the TCA cycle (e.g. valine, isoleucine).

Thus for example in the case where the medium is lipid deficient or limited in lipids, the amount of lipids in the medium is such that fatty acid uptake is at least 10 fold, 20 fold, 50 fold or even 100 fold lower than glucose uptake in the cell.

Examples of nutrient types which may be deficient include, but are not limited to a fatty acid, triglyceride, cholesterol, monosaccharide, pyruvate, glycerol and an amino acid.

For example, if pyruvate and glycerol are deficient in the perfusion medium, the metabolic pathways into glycolysis are eliminated (for example pathway 75, 7 and 8 of Table 1 herein below). This is useful for determining the amount of glycolysis of the cell, as further described herein below.

For example if lipids such as fatty acids and triglycerides are deficient in the perfusion medium, the lipid oxidation pathway is eliminated. This is useful for determining the amount of glycolysis, oxidative phosphorylation, and/or glutaminolysis of the cells.

FIGs. 11A-C illustrate how when particular components are deficient in the perfusion medium, particular metabolic flux may be determined.

For example, the constituents of the perfusion medium can be selected which induce or inhibit glycolytic flux. Then, measurements of oxygen, glucose and/or lactate can be made as further detailed hereinabove and used for determining glycolytic capacity, glycolytic reserve and/or non-glycolytic acidification. In a representative example, which is not to be considered as limiting, non-glycolytic acidification is determined before perfusion of the environment altering constituents, glycolytic capacity can be determined following perfusion with saturating concentration of glucose, which is used by the cells through the glycolytic pathway to produce ATP, NADH, water, and protons, and glycolytic reserve is determined following perfusion with glycolysis inhibitor, such as, but not limited to, 2-deoxyglucose, which inhibits glycolysis by binding to hexokinase.

Exemplary metabolic flux (e.g. the rate of turnover of molecules through a particular metabolic pathway), that may be calculated using the system described herein are set forth in Table 1 herein below.

**Table 1**

| No. | Metabolic flux |
|---|---|
| 1 | Glucose + Pᵢ ↔ Glucose 6-P +H₂O |
| 2 | Glucose 6-P ↔ Fructose 6-P |
| 3 | Fructose 6-P + Pi ↔ Fructose 1,6-P₂ +H₂O |
| 4 | Fructose 1,6-P₂ ↔ 2 Glyceraldehyde 3-P |
| 5 | Glyceraldehyde 3-P + NAD⁺ + Pi⁺ ADP ↔ Phosphoenolpyruvate + NADH + H⁺ + ATP |
| 6 | Phosphoenolpyruvate + ADP → Pyruvate + ATP |
| 7 | Pyruvate + CoA + NAD⁺ → Acetyl-CoA + CO₂ + NADH |
| 8 | Lactate + NAD⁺ ↔ Pyruvate + NADH + H⁺ |
| 9 | Acetyl-CoA + Oxaloacetate + H₂O→ Citrate + CoA + H⁺ |
| 10 | Citrate + NAD+ ↔ 2-oxo-gluterate + NADH + CO₂ |
| 11 | 2-oxo-gluterate + NAD⁺ + CoA → Succinyl-CoA + NADH + CO₂ +H⁺ |
| 12 | Succinyl-CoA + Pᵢ+ GDH + FDH ↔ Fumarate + GTP + FADH₂ +CoA |
| 13 | Fumarate + H₂O ↔ Malate |
| 14 | Malate + NAD+ ↔ Oxaloacetate + NADH + H⁺ |
| 15 | Arginine + H₂O → Ornithine + Urea |
| 16 | Ornithine + CO₂ + NH₄⁺ + 2 ATP + H₂O ↔ Citrulline + 2 ATP + 2Pi + 3H⁺ |
| 17 | Citrulline + Aspartate + ATP → Arginine + Fumarate + AMP + PPi |
| 18 | Arginine uptake |
| 19 | Ammonia Uptake |
| 20 | Ornithine Output |
| 21 | Alanine + 0.5 NAD⁺ + 0.05 NADP⁺ + H₂O → Pyruvate + NH₄⁺ + 0.5 NADH + 0.5 NADPH + H⁺ |
| 22 | Alanine Uptake |
| 23 | Serine → Pyruvate + NH₄⁺ |
| 24 | Serine Uptake |
| 25 | Cysteine + 0.5 NAD⁺ + 0.5 NADP⁺ +H₂O + SO₃ ²⁻ → Pyruvate + Thiosulfate + NH₄⁺ + 0.5 NADH + 0.5 NADPH + H⁺ |
| 26 | Cysteine Uptake |
| 27 | Threonine + NAD⁺ → Glycine + Acetyl-CoA + NADH |
| 28 | Glycine + NAD⁺ + H₄folate ↔ N⁵, N¹⁰-CH₂H₄folate + NADH + CO₂ + NH₄⁺ + H⁺ |
| 29 | Glycine Uptake |
| 30 | Tryptophan + 3 H₂O + 3 O₂ + CoA + 3 NAD⁺ + FAD → 3CO₂ + FADH₂ + 3NADH + 4H⁺ + NH₄⁺ + Acetoacetatyl-CoA |
| 31 | Propionyl-CoA + CO₂ + ATP → Succinyl-CoA + AMP + PPi |
| 32 | Lysine + 3H₂O + 5 NAD⁺ + FAD + CoA → 2 NH₄⁺ + 5 NADH + 5 H⁺ + FADH₂ + 2 CO₂ + Acetoacetatyl-CoA |
| 33 | Phenylalanine + H₄biopterin + O₂ → Tyrosine + H₂biopterin + H₂O |
| 34 | Tyrosine + 0.5 NAD⁺ + 0.5 NADP⁺ + H₂O + 2 O₂ → NH₄⁺ + CO₂ + 0.5 NADH + 0.5 NADPH + H⁺ + Fumarate + Acetoacetate |
| 35 | Tyrosine Uptake |
| 36 | Glutamate + 0.5 NAD⁺ + 0.5 NADP⁺ + H₂O ↔ NH₄⁺ + 2-oxo-gluterate + 0.5 NADH + 0.5 NADPH + H⁺ |
| 37 | Glutamate Uptake |
| 38 | Glutamine + H₂O → Glutamate + NH₄⁺ |
| 39 | Ornithine + NAD⁺ + NADP⁺ + H₂O → Glutamate + NH₄⁺ + NADH + NADPH + H⁺ |
| 40 | Proline +0.5 O₂ + 0.5 NAD⁺ + 0.5 NADP⁺→ Glutamate + 0.5 NADH + 0.5 NADPH + H⁺ |
| 41 | Histidine+ H₄folate + 2H₂O → NH₄⁺ + N5-formiminoH₄folate + Glutamate |
| 42 | Methionine + ATP + Serine + NAD⁺ + CoA → PPi + Pi + Adensosine + Cysteine + NADH + Propionyl-CoA + CO₂ + NH₄⁺ |
| 43 | Aspartate + 0.5 NAD⁺ + 0.5 NADP⁺ + H₂O ↔ Oxaloacetate + NH₄⁺ + 0.5 NADH + 0.5 NADPH + H⁺ |
| 44 | Aspartate Uptake |
| 45 | Asparagine + H₂O → Aspartate + NH₄⁺ |
| 46 | 8 Acetyl-CoA + 7 ATP + 14 NADPH + 14 H⁺ → Palmitate + 8 CoA + 6 H₂O + 7 ADP + 7Pᵢ + 14 NADP⁺ |
| 47 | 2 Acetyl-CoA ↔ Acetoacetyl-CoA + CoA |
| 48 | Acetoacetyl-CoA + H₂O → Acetoacetate + CoA |
| 49 | Acetoacetate Output |
| 50 | Acetoacetate + NADH + H⁺ ↔ β-hydroxybutyrate + NAD⁺ |
| 51 | NADH + H⁺ + 0.5 O₂ + 3 ADP → NAD⁺ + H₂O + 3 ATP |
| 52 | FADH₂ + 0.5 O₂ + 2 ADP → FAD + H₂O + 2 ATP |
| 53 | O₂ Uptake |
| 54 | Glucose 6-P + 12 NADP⁺ + 7 H₂O → 6 CO₂ + 12 NADPH + 12 H⁺ + Pᵢ |
| 55 | Valine + 0.5 NADP⁺ + CoA + 2 H₂O + 3.5 NAD⁺ + FAD → NH₄⁺ + Propionyl-CoA + 3.5 NADH + 0.5 NADPH + 3 H⁺ +FADH₂ + 2 CO₂ |
| 56 | Isoleucine + 0.5 NADP⁺ + H₂O + 2.5 NAD⁺ + FAD + 2 CoA → NH₄⁺ + Propionyl-CoA + Acetyl-CoA + 2.5 NADH + 0.5 NADPH + 3H⁺ + FADH₂ +CO₂ |
| 57 | Leucine + 0.5 NADP⁺ + H₂O + 1.5 NAD⁺ + FAD + ATP + CoA → NH₄⁺ +1.5 NADH + 0.5 NADPH + 2 H⁺ + FADH₂ + ADP + Pᵢ + Acetoacetate + Acetyl-CoA |
| 58 | Threonine uptake |
| 59 | Lysine Uptake |
| 60 | Phenylalanine Uptake |
| 61 | Glutamine Uptake |
| 62 | Proline Uptake |
| 63 | Histidine Uptake |
| 64 | Methionine Uptake |
| 65 | Asparagine Uptake |
| 66 | Valine Uptake |
| 67 | Isoleucine Uptake |
| 68 | Leucine Uptake |
| 69 | Albumin Synthesis |
| 70 | Triglyceride ↔ Glycerol + 3 Palmitate |
| 71 | Triglyceride Uptake |
| 72 | Glycerol Uptake |
| 73 | Palmitate Uptake |
| 74 | Glucose-6-P + UTP + H₂O ↔ Glycogen + 2 Pi + UDP |
| 75 | Glycerol + NAD⁺ ↔ Glyceraldehyde 3-P + NADH + H⁺ |
| 76 | 18 Acetyl-CoA + 25 NADPH + NADH + 26 H⁺ +18 ATP + 11 O₂ → Cholesterol + 25 NADP⁺ + NAD⁺ + 18 ADP + 6 Pi + PPᵢ + 8 CO₂ + 6 H₂O + 18 CoA + HCOOH |
| 77 | Cholesterol + 5 NADPH + H⁺ + 3 O₂ + ATP + 2 CoA + FAD ↔ Choloyl-CoA + 5 NADP⁺ + 2 H₂O + ADP + PPᵢ + FADH₂ + Propionil-CoA |
| 78 | Cholesterol Output |
| 79 | Bile Output |
| 80 | CO₂ output |

The method of the present embodiments are not being part of the invention as claimed in claim 1, but can be used to determine mitochondrial function, such as, but not limited to, basal respiration, ATP-linked respiration, H⁺ leak, maximal respiration and spare respiratory capacity. For example, modulators of cellular respiration that specifically target components of the electron transport chain can be perfused to the plate. Then, measurements of oxygen, glucose and/or lactate can be made as further detailed hereinabove and used for determining ATP-linked respiration, maximal respiration, and also non-mitochondrial respiration. Proton leak and spare respiratory capacity can also be calculated using the basal, ATP-linked, maximal, and non-mitochondrial respiration. In a representative example, which is not to be considered as limiting, basal respiration is determined before perfusion of the modulator, ATP-linked respiration is determined following perfusion of oligomycin, maximal respiration can be determined following perfusion of FCCP, and non-mitochondrial respiration can be determined following perfusion of rotenone and antimycin A.

The method of the present embodiments are not being part of the invention as claimed in claim 1, but can be used to determine one or more parameters indicative of glycolytic flux, such as, but not limited to, is glycolytic capacity, glycolytic reserve, and non-glycolytic acidification.

In some embodiments not being part of the invention as claimed in claim 1, the delivery and analysis is repeated. For example, sequential measurements of a single group of cells may be made at predetermined time intervals to analyze the effect of a compound addition temporally, for example to examine the effect of exposure to a drug, chemical, or toxin.

The measurement of the oxygen and lactate/glucose is typically effected for at least one hour, two hours, 6 hours, 12 hours, 24 hours, 2 days, 1 week, 2 weeks, 3 weeks, 4 weeks or longer.

Preferably, the analysis of oxygen and glucose consumption and lactate production is effected once the cells have reached a steady-state.

In one embodiment not being part of the invention as claimed in claim 1, but, the steady state of the cells refers to a constant uptake of glucose and oxygen which does not change with culture time. In one embodiment not being part of the invention as claimed in claim 1" the measurement of oxygen is continuous (i.e. effected in real-time). Measurement of lactate and/or oxygen may be effected at regular intervals during the same time period, for example once every 10 minutes throughout the time period that the oxygen is measured, once every 30 minutes throughout the time period that the oxygen is measured, once an hour throughout the time period that the oxygen is measured etc. Of note, the analysis is such that the cells remain viable throughout the time period.

The system described herein can be used to measure metabolic flux of cells over an extended period of time.

The system may be used to study the effect of agents on the metabolic flux of the cells and comparing the metabolic flux of cells using the system described herein in the presence and absence of those agents.

Exemplary agents that may be analyzed include pharmaceutical agents, agricultural agents, cosmetic agents and diagnostic agents. The agent may be a drug candidate. The agent may be a small molecule, a polypeptide or a polynucleotide.

As used herein the term "about" refers to ± 10 %.

The word "exemplary" is used herein to mean "serving as an example, instance or illustration." Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments." Any particular embodiment of the invention may include a plurality of "optional" features unless such features conflict.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects as delineated hereinabove and as claimed in the claims section below find experimental support in the following examples.

### EXAMPLES

Microfluidic organ-on-a-chip technology is poised to replace animal toxicity testing, but thus far demonstrated few advantages over traditional methods. In the present Example the Inventors demonstrate a sensor-integrated platform permitting fast tracking of the dynamics of metabolic adaptation to mitochondrial dysfunction. The approach described herein permits detection of chemical toxicity before any effects on cell or tissue viability can be observed.

Using a lung-on-chip device, it was demonstrated that mechanical forces are critical for the development of nanoparticle toxicity in lung tissue [1], and that long-term maintenance of hepatocyte function can be obtained in a microfluidic bioreactor [2-5]. Regretfully, most organ-on-chip devices still rely on endpoint assays to assess drug toxicity, providing limited dynamic information that can be used to assess the compound mechanism of action [6, 7].

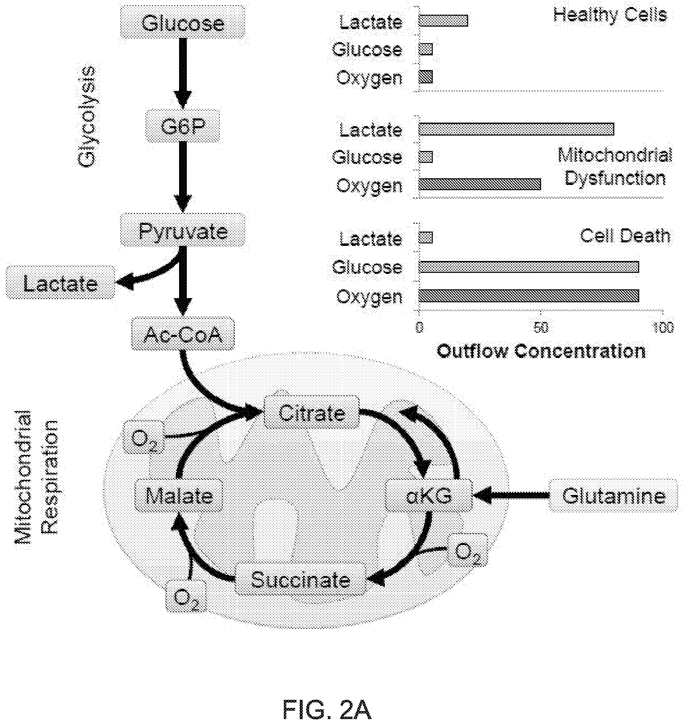

Mitochondrial function plays a role in the development of disease and chemical toxicity [7, 8], as well as the regulation of stem cell pluripotency [9]. Mitochondrial dysfunction can occur through direct damage to the respiratory chain, or through secondary mechanisms such as ER stress or lipid accumulation [10]. As cells can adapt to loss of mitochondrial respiration by utilizing anaerobic pathways such as glycolysis and glutaminolysis (FIG. 2A), mitochondrial damage can often go undetected with disastrous clinical and financial consequences (8). This is a particular concern in cultured cells that show an increased reliance on glycolysis and glutaminolysis due to the Warburg effect. Thus, fast monitoring of these metabolic fluxes could provide critical insight into the development of mitochondrial dysfunction, as well as loss and gain of mitochondrial function in development.

Recently, fast measurement of oxygen uptake in liver-on-chip devices was demonstrated using two-frequency phase modulation of tissue-embedded phosphorescent microprobes [11]. Oxygen decreases phosphorescence decay time resulting in a phase change that is independent of signal intensity. In contrast to other methods, phase modulation is unaffected by focus distance or changing microprobe concentration due to cell death or migration, thus providing unparalleled ability to monitor oxygen uptake in real-time.

### EXAMPLE 1

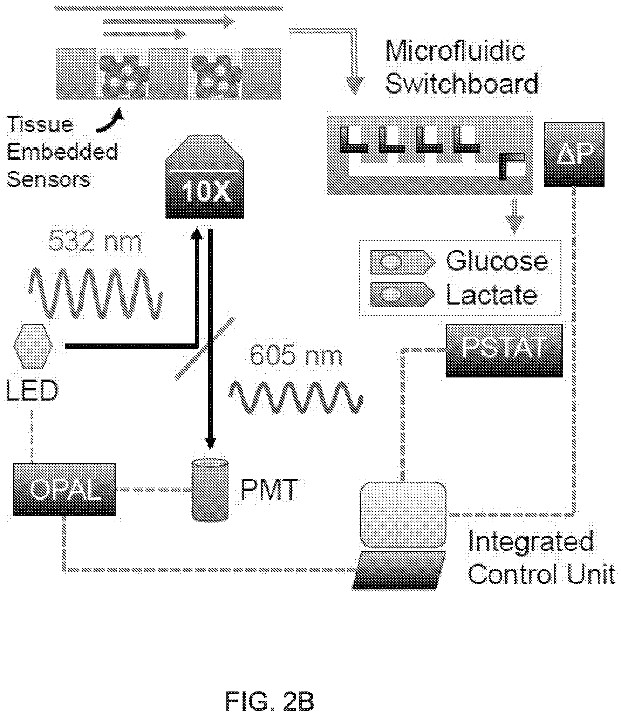

The design presented in this disclosure offers an advantage over Clark-type electrodes that consume oxygen and require precise positioning and frequent recalibration [12]. It is appreciated by the inventors that oxygen measurements provide only circumstantial evidence to loss of mitochondrial function. This Example describes a device capable of maintaining three-dimensional aggregates of HepG2/C3A cells for over 28 days *in vitro* under physiological shear forces and stable oxygen gradient mimicking the native liver microenvironment. Oxygen uptake rate is monitored in real-time using two-frequency phase modulation, while a computer controlled microfluidic switchboard automates washing, calibration and amperometric measurements of glucose uptake and lactate production over the same time period (FIG. 2B). The system described herein permits the detection of minute shifts from oxidative phosphorylation to glycolysis or glutaminolysis, indicating mitochondrial damage in drug concentrations previously believed to be safe. The measurements optionally and preferably permit analysis of the redistribution of ATP production in cells undergoing mitochondrial stress and dysfunction, unraveling the dynamics of the metabolic adaptation to mitochondrial damage.

### System Design

FIG. 2A is a schematic of glucose and glutamine utilization by central carbon metabolism. Theoretically, mitochondrial dysfunction leads to a decrease in oxygen uptake and increase in lactate production due to cellular shift from oxidative phosphorylation (purple) to glycolysis (orange).

FIG. 2B is a schematic illustration of the measurement setup. Bioreactor was loaded with tissue-embedded oxygen sensors and mounted on an Olympus IX83. OPAL unit controlled LED signal modulation, exciting the oxygen sensors, and analyzed the signal through the photomultiplier (PMT) read-out. Bioreactor outflow was connected to a microfluidic switchboard containing a series of pressure-controlled micromechanical valves. The switchboard introduced samples into a sensor unit containing electrochemical sensors for glucose and lactate. Sensors were controlled by a potentiostat (PSTAT). Optical, pressure, and electronic controller were connected to a single microprocessor the synchronized the signal.

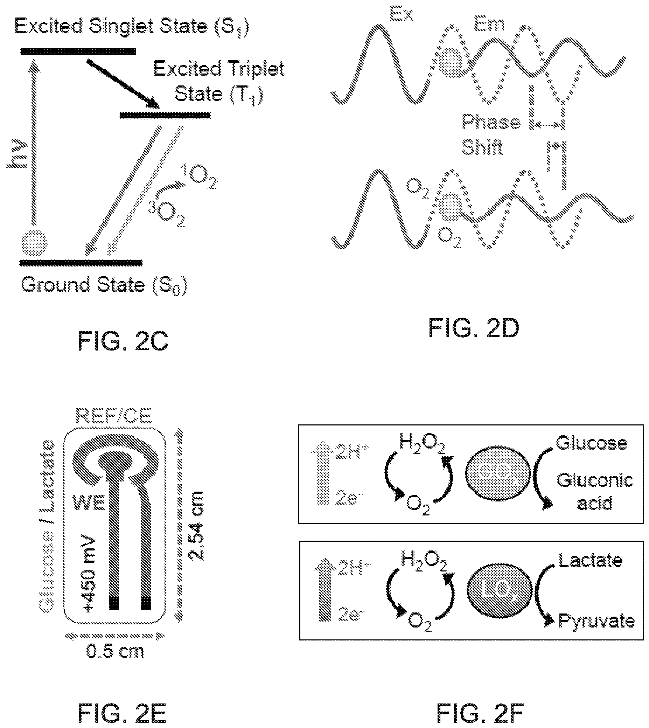

FIG. 2C is a Jablonski diagram describing the generation of phosphorescence with Ru-CPOx beads under the influence of oxygen. The quenching of the phosphorescence by triplet oxygen leads to a decrease in signal intensity and phosphorescence decay time (T1).

FIG. 2D demonstrates that the effect described in FIG. 2C induces a phase shift between the intensity-modulated excitation and emission light. Thus, the degree of phase shift can be used for determining the oxygen concentration. Two-superimposed frequencies were used to screen out background interference.

FIG. 2E schematically illustrates a design of amperometric sensor in 2-electrode configuration. Anodic oxidation of H2O2 on the platinum working-electrode (WE) held at 450 mV against the reference electrode (REF/CE) produces a detected current.

FIG. 2F is a schematic illustration describing that that H₂O₂ is created in equivalent amounts of the analyte as an intermediate product by the activity of glucose oxidase (GOx) or lactate oxidase (LOx).

Mitochondrial dysfunction is a major cause of drug-induced injury due to the limited ability of cells to generate ATP by alternatives means. Increasing glycolysis, fermentation of glucose to lactate, is the preferred route of energy production in the absence of oxygen (FIG. 2A). Glutamine degradation to lactate and citrate, termed glutaminolysis, can also support tumor cell growth under hypoxic conditions. Mitochondrial damage can lead to rapid compensation, as cells shift from mitochondrial respiration to glycolysis to survive. To monitor the dynamic transition between these metabolic pathways a microfluidic system that maintains growth-arrested HepG2/C3A spheroids under physiological conditions while dynamically measuring oxygen, glucose, and lactate concentrations was designed (FIG. 2B).

Oxygen is measured using tissue-embedded microparticles loaded with a ruthenium-based dye, whose phosphorescence is quenched in the presence of oxygen leading to decreasing decay time (FIG. 2C). In contrast to intensity measurement, decay time is insensitive to probe concentration or excitation intensity. The present Example uses sinusoidal intensity-modulated light, resulting in an oxygen-dependent phase shift in the 605 nm emission (FIG. 2D). Using this system, signal is stable down to 3 particles, and 1.5 mm away from the microscope focus [11]. In contrast to optical measurements, electrochemical sensors require frequent recalibration and demonstrate significant decay over time.

In the present Example, off-the-shelf medical-grade sensors were integrated in the microfluidic design, and were addressed using a computer-controlled microfluidic switchboard that sequentially samples the bioreactor outflow, washes and recalibrates the sensors. The amperometric glucose and lactate sensors use platinum electrodes to measure current under a polarization of 450 mV (FIG. 2E). Current is produced by the oxidation of glucose or lactate to H₂O₂ by membrane-embedded glucose or lactate oxidase (FIG. 2F). A single CPU controls the entire system, simplifying synchronization [13].

### Design and Characterization of Hepatic Microwell Bioreactor

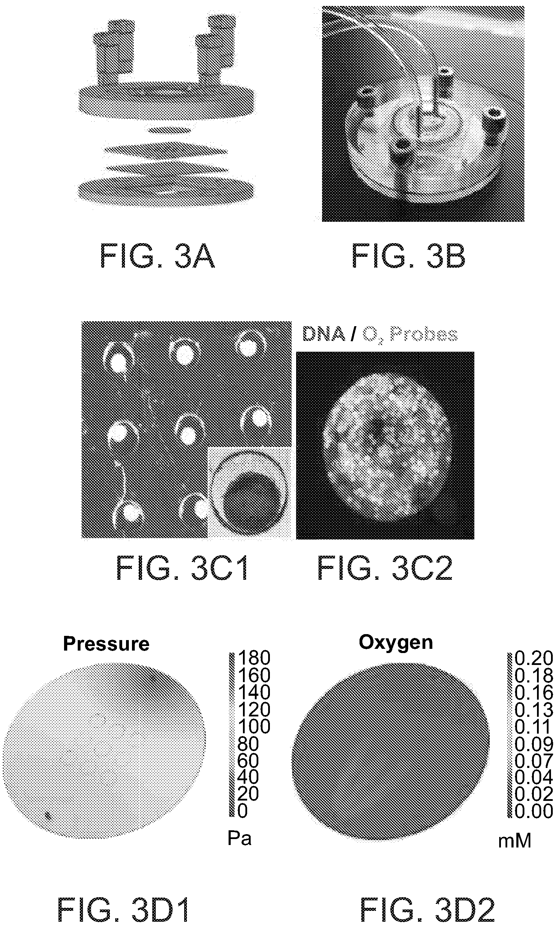

FIG. 3A is a schematic explosive view of a bioreactor according to some embodiments of the present invention. From bottom: PMMA housing, coverglass, laser-cut PDMS microwells, top glass window, and PMMA cover.

FIG. 3B is an image of assembled bioreactor on microscope stage.

FIGs. 3C1 and 3C2 demonstrate HepG2/C3A organoid after overnight incubation with embedded oxygensensing micro-probes (orange).

FIGs. 3D1 and 3D2 show results of numerical simulation of the pressure drop and variations in oxygen concentration throughout the bioreactor.

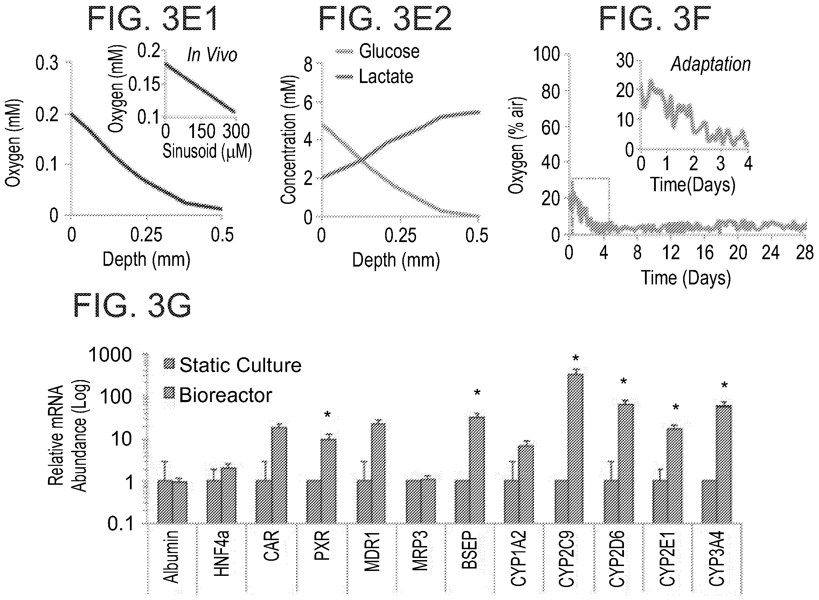

FIGs. 3E1 and 3E2 show cross-section of oxygen gradient developing due to consumption within the well (from top to bottom), mimicking the *in vivo* microenvironment. Gradients of glucose and lactate concentrations within the well are shown on the right.

FIG. 3F shows representative longterm oxygen measurement over 1 month in bioreactor perfused with cell culture medium at 2 µl/min. 100% air represents atmospheric dissolved oxygen concentration (no consumption). Cultures reach steady state within 4 days after seeding.

FIG. 3G shows log-scale quantitative gene expression analysis of HepG2/C3A cells in static culture compared to those perfused with 1% DMSO for 30 days (growth arrest). PXR, CYP3A4 and CYP2C9 expression shows a 10, 58, and 300-fold increase in the bioreactor, respectively (p<0.024, n=3). Values are within 10% to 30% of primary human hepatocytes. (^{∗} p < 0.05 by student's T-test).

The liver is highly vascularized, delivering oxygen at high rates while protecting hepatocytes from the negative effects of shear [14, 15]. The gradients that develop along the sinusoid induce a demarcation of function, termed metabolic zonation. To mimic this environment a PMMA bioreactor with a detachable multiwell unit that permits cell seeding in an open configuration was designed (FIGs. 3A and 3B).

HepG2/C3A cells mixed with oxygen probes were seeded in the microwells protecting the cells from the negative effect of shear forces while providing a constant concentration of nutrients and removal of metabolic waste products. Cells aggregate to spheroids after overnight incubation (FIGs. 3C1-3C2), polarizing and reaching growth arrest in 4 to 7 days in the presence of 1% DMSO [16, 17]. Computational fluid dynamic modeling (FIGs. 3D1, 3D2, 3E1 and 3E2) showed physiological shear forces under 0.03 Pa inside the microwells for perfusion rates of 2 µl/min. The flow rate resulted in similar oxygen concentration delivered to each well in the array (FIGs. 3D1, 3D2, 3E1 and 3E2). Oxygen consumption caused a gradient to develop along the perfused spheroid, mimicking the in vivo microenvironment (FIGs. 3E1 and 3E2) [18, 19]. Glucose concentration showed a similar gradient, reaching 0.1 mM concentration at the bottom of the microwells. These results suggest that the reactor of the present embodiments mimics aspects of the physiological microenvironment.

Oxygen uptake was measured in real-time over 28 days under continuous perfusion (FIG. 3F). Measurement showed an increase in oxygen uptake, stabilizing on day 4, suggesting the cells reached steady state. Gene expression on day 30 of culture showed marked elevation of liver specific gene expression compared to static culture (FIG. 3G). Primarily, expression of the Pregnane X receptor (PXR) and the constitutive androstane receptor (CAR) increase by 10 and 18-folds, respectively (p<0.024, n=3), while their target genes MDR1, CYP2C9 and CYP3A4 showed a 20- to 300-folds increase in mRNA abundance (p<0.022, n=3).

### Real-Time Oxygen Measurement shows Rapid Loss of Mitochondrial Respiration

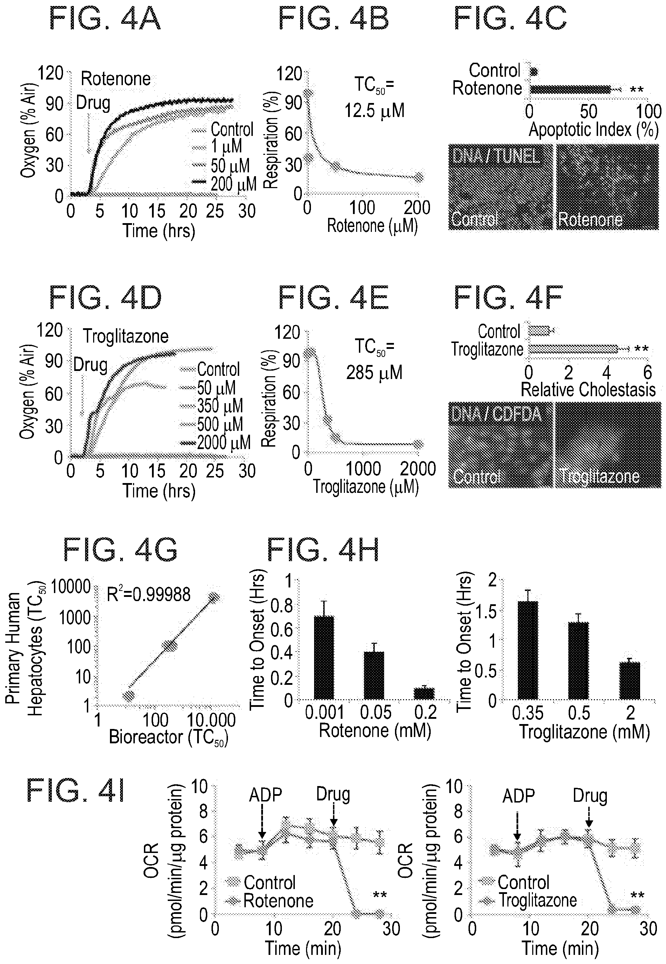

FIG. 4A shows representative oxygen uptake over time response of HepG2/C3A cells exposed to increasing concentrations of rotenone.

FIG. 4B shows dose dependence of rotenone after 12 hours. TC50 was calculated to be 12.5 µM.

FIG. 4C demonstrates that TUNEL^{™} staining shows 14-fold increase in apoptosis following 24 hours exposure to 200 µM rotenone (p<0.001, n=5), as well as unlabeled cell death suggesting necrosis.

FIG. 4D shows representative oxygen uptake over time response of HepG2/C3A cells exposed to increasing concentrations of troglitazone.

FIG. 4E shows dose dependence of troglitazone after 24 hours. TC50 was calculated to be 285 µM.

FIG. 4F demonstrates that CDFDA staining shows 4.5-fold increase in intracellular accumulation of bile acids (cholestasis) following 24 hours exposure to 200 µM troglitazone (p<0.001, n=5).

FIG. 4G is a IVIVC curve comparing TC50 values of primary human hepatocytes to the bioreactor showing an excellent R2=0.99 correlation for acetaminophen, amiodarone, troglitazone, and rotenone.

FIG. 4H demonstrates that the time to onset of mitochondrial damage was dose-dependent for both rotenone (left) and troglitazone (right), occurring in minutes rather than hours following exposure to the drugs.

FIG. 4I shows oxygen consumption rate (OCR) measured on isolated mitochondria for 30 min, followed by ADP injection (arrow) and subsequent injection of 50 µM rotenone (left) or troglitazone (right). Loss of OCR was immediate (p<0.001, n=3) and did not require cytosolic enzyme activation of the drugs. (^{∗∗} p<0.01 by student's test).

Rotenone is a widely used, broad-spectrum organic insecticide and pesticide, known to directly damage mitochondrial complex I, inducing oxidative stress and apoptosis at low concentrations (20). Rotenone was perfused at concentrations of 1, 50, and 200 µM for 24 hours and oxygen uptake of the cells was monitored in real-time to assess cell viability and metabolic function (FIG. 4A). Oxygen uptake dropped rapidly when cells were exposed to 1, 50, and 200 µM rotenone, reaching 35%, 27%, and 15% of normal respiration following 12 hours exposure, respectively. The majority of the effect occurred within the first 60 minutes, as expected from a direct complex I inhibitor. Plotting the dosedependent response of rotenone at 12 hours, a TC50 of 12.5 µM was calculated (FIG. 4B), compared with 2 µM in primary hepatocytes. To evaluate the toxicological end point of rotenone, the number of intact and apoptotic nuclei was quantified using the TUNEL assay (FIG. 4C). Cells exposed to rotenone showed a 14±2 fold increase in apoptosis (p<0.001, n=5), as well as unlabeled cell death suggesting necrosis.

Troglitazone is an anti-diabetic and anti-inflammatory drug which was removed from market due to severe drug-induced liver injury [21]. While troglitazone mechanism of action is still unclear, its metabolites were shown to block bile acid transporters BSEP and MRP2 leading to cholestasis, while the parent compound was shown to decrease mitochondrial membrane potential [22]. Troglitazone was perfused at concentrations ranging from 50 to 2000 µM for 24 hours and oxygen uptake of the cells was monitored in real-time to assess cell viability and metabolic function (FIG. 4D). Oxygen concentration at 50 µM troglitazone was not significantly different from control. However, oxygen uptake dropped when cells were exposed to 350, 500, and 2000 µM troglitazone, reaching 32%, 15% and 8% of normal respiration following 24 hours exposure, respectively. Interestingly, most of the effect occurred within the first 5 hours, suggesting direct mitochondrial damage rather than accumulation of secondary metabolites [11]. Plotting the dose-dependent response of troglitazone at 24 hours, a TC50 of 285 µM was calculated (FIG. 4E), compared with 100 µM in primary hepatocytes. To evaluate the toxicological end point of troglitazone, CDF retention in treated and untreated cells was evaluated (FIG. 4F). Cells exposed to troglitazone showed a 4.5±0.6 fold increase in CDF staining due to inhibition of MRP2 (p<0.001, n=5).

Overall, comparison of the results to primary human hepatocytes (IVIVC) across 4 drugs, including acetaminophen, amiodarone, troglitazone, and rotenone showed an R2 of 0.99 (FIG. 4G). Both rotenone and troglitazone showed a dose-dependent time to onset of damage (FIG. 4H). Rotenone acted within 6 minutes of administration at 0.2 mM, while troglitazone showed respiratory damage within 40 to 100 minutes. The dynamics of these responses suggest that both compounds directly damage the mitochondria, rather than produce a slow accumulation of toxic intermediates. To evaluate this, oxygen consumption rates (OCR) of ADP-stimulated isolated mitochondria were measured (FIG. 4I). Troglitazone, like rotenone, can directly inhibit mitochondrial respiration in minutes (p<0.001, n=3) in the absence of enzymatic activation.

### Automated Microfluidic Measurement of Glucose and Lactate

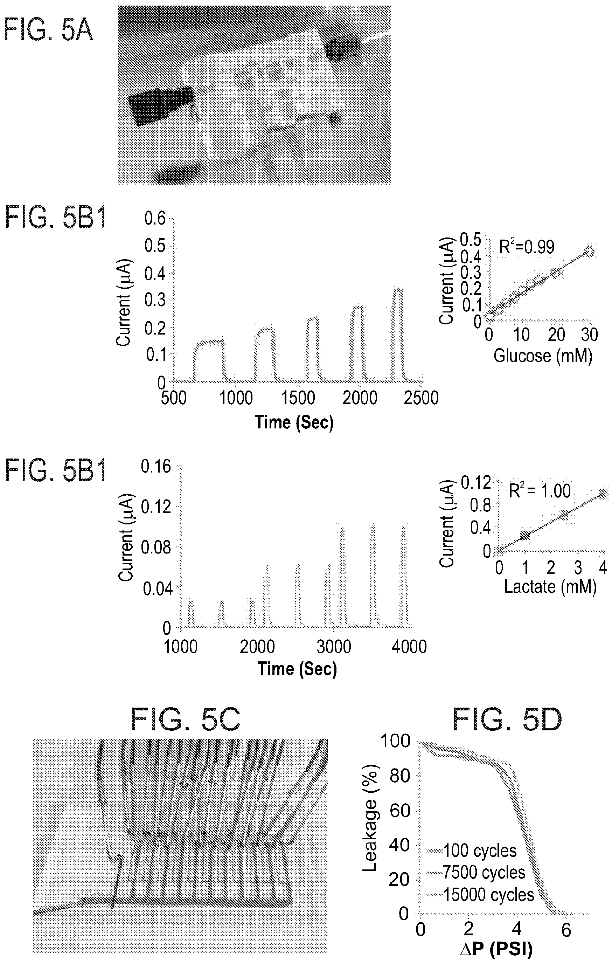

FIG. 5A is an image of PMMA unit housing both glucose and lactate sensors with total internal volume of 26 µL.

FIGs. 5B1 and 5B2 show amperometric calibration curves of glucose (FIG. 5B1) and lactate (FIG. 5B2) sensors in the PMMA housing. Measurements were carried out under static condition for 100 to 200 seconds. Air purging before sample introduction insured sharp change in chemical gradient.

FIG. 5C is an image of two-layer microfluidic switchboard containing flow channels (red) and independently addressed control channels (blue).

FIG. 5D shows characterization of micromechanical valve switching pressure as a function of number of cycles (age). Valves withstand over 15,000 cycles without loss of sensitivity, up to 300 days of continuous operation.

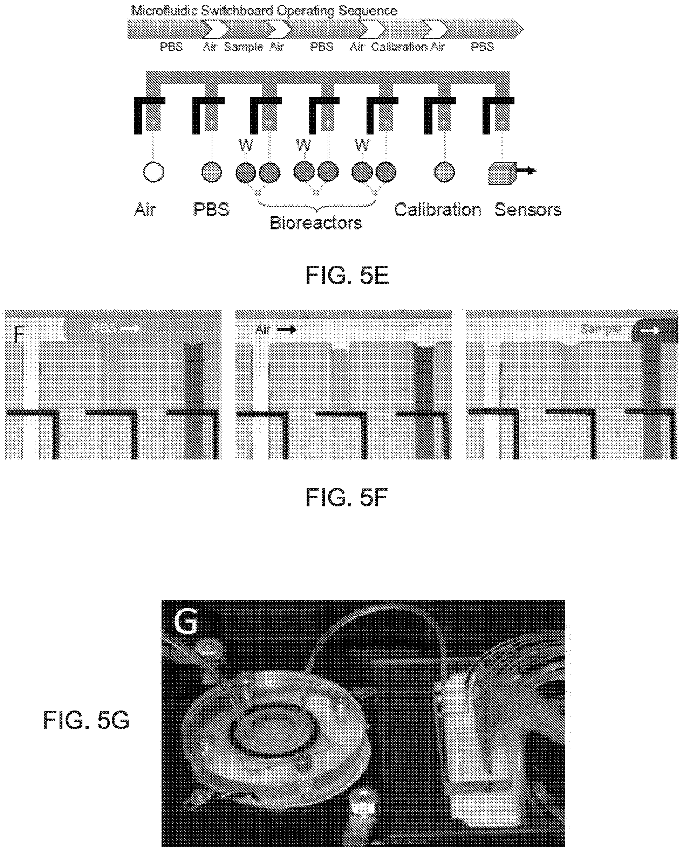

FIG. 5E is a schematic illustration of microfluidic switchboard connectivity and operating sequence. Switchboard contained inputs to air, washing buffer (PBS), and calibration solution. Bioreactors outflow was split to high-resistance waste (W) and normally closed channel to switchboard. Air purging was carried out for 4 second before and after measurement or calibration step. 200 seconds of washing intersected between measurements.

FIG. 5F shows images of perfusion sequence starting with a washing step (blue), air purging, and sample introduction (red).

FIG. 5G is an image of a simplified set-up where a single bioreactor is connected to the switchboard.

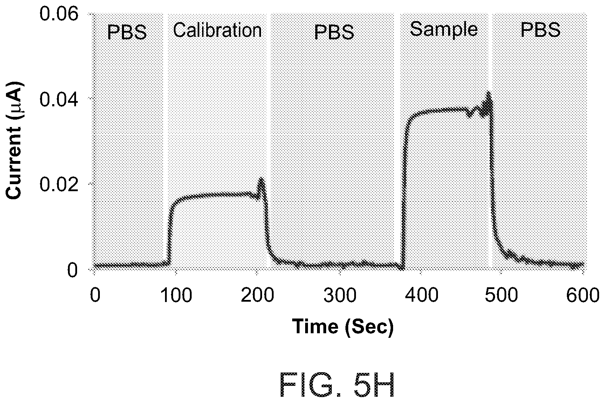

FIG. 5H shows automatic amperometric calibration and measurement of glucose concentration in bioreactor outflow.

An off-chip sensor unit (FIG. 5A) containing off-the-shelf medical-grade sensors was fabricated. Accurate measurement required a sharp chemical gradient, which was achieved by purging the system with air before each measurement, producing a linear response of glucose and lactate sensors up to 20 mM (FIGs. 5B1 and 5B2). To carry out the experimental steps automatically, a microfluidic switchboard containing 12 self-addressable micro-mechanical valves (FIG. 5C) connected to a 32-bit ARM7-based control unit that synchronizes flow, measurement and stimulation [13] was fabricated. Micro-mechanical valves were fabricated using two-layer soft lithography showing an actuation pressure of 4 PSI and stability for over 15,000 cycles, 150 to 300 days at the sampling rate (FIG. 5D). Bioreactors were continuously perfused with their outflow split into a high resistance waste and the valve-controlled switchboard (FIG. 5E). Sample was obtained every hour by opening the valve for 20 min and permitting a 40 µL sample to be introduced into the sensor unit. Sampling was preceded and followed by a 4 seconds purging with air (FIGs. 5E and 5F) creating a sharp concentration step-change. 200 seconds washing step was intertwined between sampling and calibration steps. The entire sequence was 23.3 minutes long, permitting a measurement of 3 bioreactors per hour.

At steady state, glucose and lactate concentration at the bioreactor outflow were 2.9 mM and 5 mM, respectively (FIGs. 5G and 5H). Cell number was quantified by DNA content to be 10⁵ cells/well resulting in glucose uptake rate of 2.4×10⁻⁹ mol/min/10⁶ cells and lactate production rate of 3×10⁻⁹ mol/min/10⁶ cells under steady state conditions. Each mole of glucose can produce 2 moles of lactate in anaerobic glycolysis, or utilize 6 moles of oxygen in oxidative phosphorylation (FIG. 2A). The results show a lactate over glucose ratio of 1.3 for the cells, suggesting that 62% of glucose is used in glycolysis. Together with the oxygen measurements of the present embodiments (FIGs. 3A-G) it is suggested that 13% of glucose consumed is directed toward oxidative phosphorylation with 26% directed toward other metabolic pathways such as lipogenesis. To exclude fatty acid oxidation their uptake rate was measured in the bioreactor outflow. Fatty acid uptake was 4.8×10⁻¹¹ mol/min/10⁶ cells; about 50-fold lower than glucose uptake.

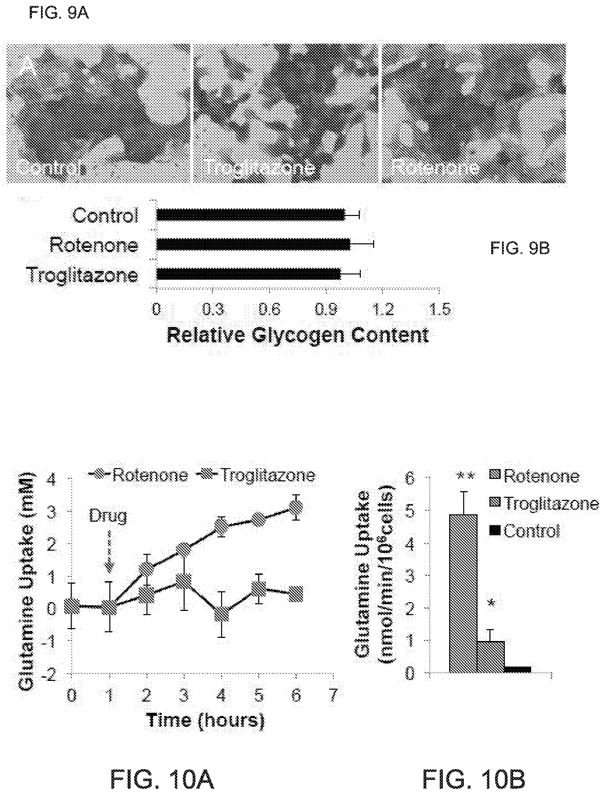

FIG. 10A shows glutamine uptake (outlet-inlet) in HepG2/C3A bioreactors exposed to 50 µM troglitazone or rotenone (dotted arrow). Rotenone exposure caused an increase in glutamine uptake while troglitazone increase was more muted.

FIG. 10B shows glutamine uptake rate, averaged over 3-6 hours, during exposure to troglitazone, rotenone, or control.

Glutamine uptake was 1.6×10⁻¹⁰ mol/min/10⁶ cells; 10-fold lower than glucose uptake, suggesting glutaminolysis had a minor contribution to the mitochondrial Krebs cycle, which was predominantly utilizing glucose as a source of energy in steady state (FIG. 2A). It is noted that proliferating HepG2 cells exhibit quite a different metabolic signature under static conditions, showing lactate over glucose ratio ranging from 1.8 to 2.

### Dynamics of Drug-Induced Mitochondrial Dysfunction

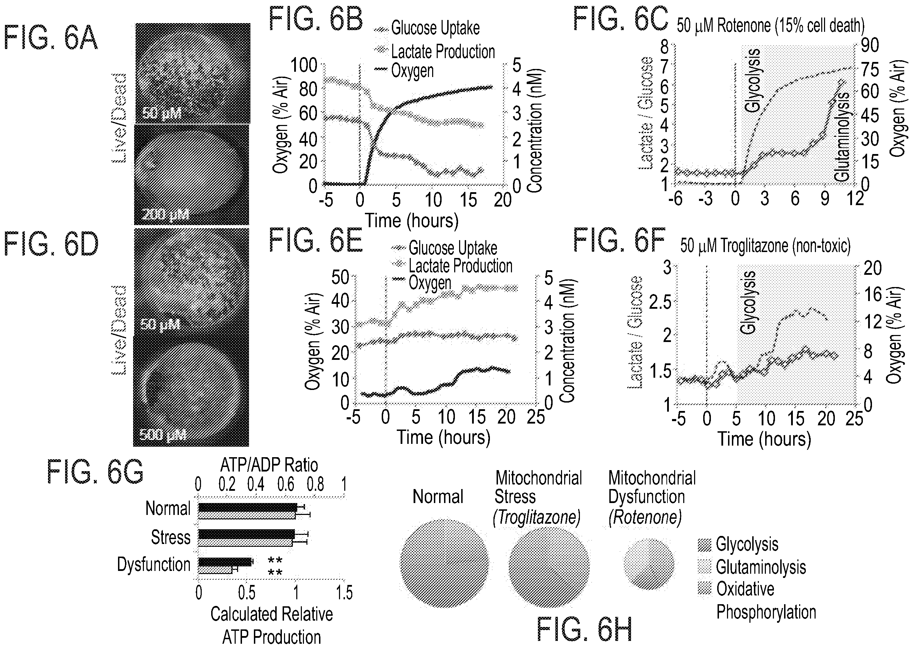

FIG. 6A shows Live/Dead staining of HepG2/C3A organoids in bioreactors treated for 24 hours with 50 or 200 µM rotenone. Approximately 15% of the cells died following exposure to 50 µM rotenone.

FIG. 6B shows representative curves of oxygen uptake, glucose uptake, and lactate production of HepG2/C3A cells following exposure to 50 µM rotenone (dotted line). Glucose uptake is shown as inlet - outlet concentration (red circles), while lactate production is shown as outlet - inlet concentration (green squares).

FIG. 6C shows changes in lactate over glucose ratio following exposure to rotenone (dotted line). Ratio shifts from 1.5 to 2.6 within 3 hours after exposure indicating a shift from oxidative phosphorylation to glycolysis. Ratio spirals to 6.1 after 6 hours exposure followed by increase in cell death.

FIG. 6D shows Live/Dead staining of HepG2/C3A organoids in bioreactors treated for 24 hours with 50 or 200 µM troglitazone. Less than 5% of the cells died following exposure to 50 µM troglitazone, not significantly different from control.

FIG. 6E shows representative curves of oxygen uptake, glucose uptake, and lactate production of HepG2/C3A cells following exposure to 50 µM troglitazone (dotted line).

FIG. 6F shows changes in lactate over glucose ratio following exposure to troglitazone (dotted line). Ratio gradually shifts from 1.3 to 1.8 within 6 hours after exposure indicating a shift from oxidative phosphorylation to glycolysis.

FIG. 6G shows relative ATP production rate calculated from flux balance analysis (Table 1, below), juxtaposed with experimentally measured ATP/ADP ratio in troglitazone, rotenone, and vehicle treated cells. Rotenone-treated cells show 47% decrease in ATP/ADP ratio (p<0.001, n=3), while troglitazonetreated cells show no significant difference in spite of the metabolic shift.

FIG. 6H shows diverging metabolic sources of ATP production in untreated cells (normal), troglitazone-treated cells (mitochondrial stress) and rotenone-treated cells (mitochondrial dysfunction) presented as pie charts of relative diameter.

Drug-induced mitochondrial dysfunction is often overlooked due to the ability of cells to circumvent damaged metabolic pathways at low drug concentrations, and secondary effects, such as apoptosis becoming dominant at increasing concentrations. To characterize the dynamics of mitochondrial dysfunction the cells were exposed to low concentrations of rotenone or troglitazone. Rotenone is a direct inhibitor of mitochondrial respiration, reducing oxygen uptake by 80% (FIGs. 4A-B) but inducing less than 15% cell death at 50 µM concentration (FIG. 6A). Real-time glucose and lactate measurements showed a sharp 59% drop in glucose uptake mirrored by a 35% drop in lactate production within the first 2 hours (FIG. 6B). Lactate over glucose ratio increased from 1.5 to 2.6 demonstrating a strong shift toward glycolysis, coupled with a rapid loss of oxygen uptake (FIG. 6C). Cellular death became apparent after 6 hours of exposure to rotenone, with an additional 65% drop in glucose uptake, but surprisingly no change in lactate production. Lactate over glucose ratio increased from 2.6 to 6.1 suggesting that glucose was no longer the main source for lactate production as the cells shifted toward glutaminolysis while drifting toward apoptosis (FIG. 4C). Indeed, offline measurement showed 30±4 folds increase in glutamine consumption (FIGs. 10A and 10B) following rotenone exposure (p<0.007, n=3).

At 50 µM concentration, troglitazone reduced oxygen uptake rate by 10% (FIGs. 4D-E), while cell viability was not significantly different from untreated control (FIG. 6D). While glucose uptake remained relatively unchanged, lactate production increased by 51% within 4 to 5 hours (FIG. 6E). Lactate over glucose ratio increase from 1.3 to 1.8 demonstrating a clear shift toward glycolysis at concentrations below the threshold of troglitazone toxicity (FIG. 6F). Offline measurement showed a mild 5±2 folds increase in glutamine consumption (FIGs. 10A-B) following troglitazone exposure (p<0.04, n=3).

### Metabolic Adaptation and Redistribution of ATP Production

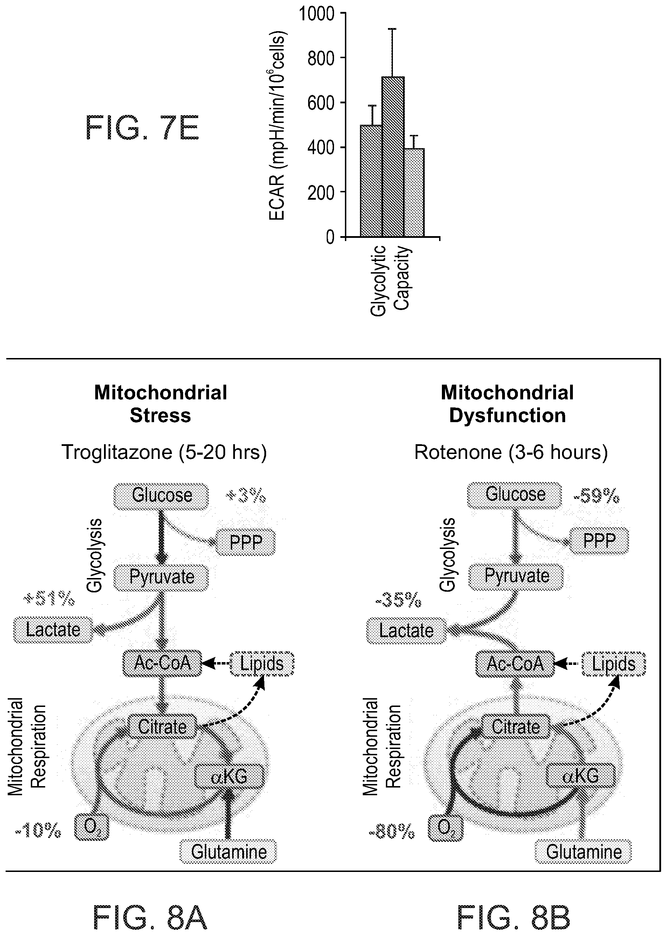

FIGs. 8A-B are schematic illustrations depicting the metabolic response of liver cells to troglitazone-induced mitochondrial stress and rotenone-induced mitochondrial dysfunction compared to untreated controls. Down-regulated fluxes are shown in green, up-regulated fluxes are shown in red. Numbers reflect measured changes in glucose, lactate, and oxygen uptake.

The results demonstrate differential metabolic response to mild or severe damage to the respiratory chain, exemplified by troglitazone and rotenone exposure, respectively (FIGs. 6A-H and 8A-B). As the flux balance stoichiometry is known (methods), the data allows estimating intracellular fluxes (Table 1, below) and predict ATP production under each condition (FIG. 6G).

Troglitazone-induced mitochondrial stress caused a 23% decrease in oxidative phosphorylation and 51% increase in glycolysis after 6 hours of exposure (Table 1). Glucose was no longer directed toward other metabolic pathways (FIGs. 8A-B) and was entirely used for ATP production (FIGs. 6G-H). These metabolic shifts, together with mild up-regulation of glutaminolysis (FIGs. 10A and 10B) allowed troglitazone-treated cells to maintain 97% of ATP production of untreated cells (FIG. 6G).

Direct measurement of ATP/ADP ratio confirms the predictions (FIG. 6G). In contrast, rotenone-induced mitochondrial dysfunction caused a 90% decrease in oxidative phosphorylation flux and 34% increase in glycolysis after 3 hours of exposure (Table 1, below). Moreover, lactate production outpaced glucose uptake suggesting glutaminolysis started playing a role in energy production, confirmed by offline measurements of glutamine uptake (FIGs. 8A-B and FIGs. 10A and 10B). In spite of these metabolic shifts, ATP production of rotenone-treated cells was less than 36% of untreated cells (FIGs. 6G-H). Direct measurement of ATP/ADP ratio confirms the predictions (p<0.001, n=3).

### Pathway Validation Under Static Conditions

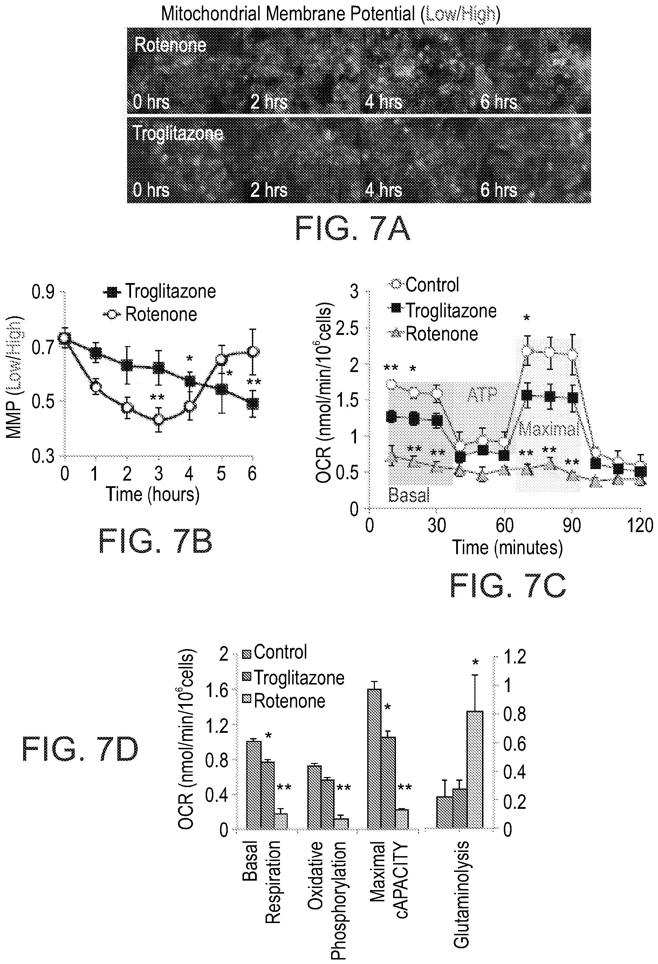

FIG. 7A shows live staining of HepG2/C3A cells with JC1 dye following exposure to 50 µM rotenone (top) or troglitazone (bottom).

FIG. 7B demonstrates that rotenone causes significant increase in mitochondrial membrane potential (MMP) after 3 hours of exposure. Rotenone causes significant increase in mitochondrial membrane potential (MMP) after 3 hours of exposure (p<0.001. n=3). Troglitazone causes a gradual increase in MMP along 6 hours of exposure (p<0.001, n=3).

FIG. 7C shows direct measurement of oxygen consumption rate (OCR) using a Seahorse XF24 analyzer, on HepG2/C3A cells exposed to 50 µM troglitazone, rotenone, or vehicle control. Basal respiration (blue) was measured for 30 min showing significant differences between the three conditions (p<0.001, n=3). Oligomycin was injected at 30 min blocking ATP production due to oxidative phosphorylation (red). FCCP was injected at 60 min, followed by complex I and III inhibitors at 90 min, showing differences in maximal mitochondrial capacity (green).

FIG. 7D demonstrates that troglitazone induced 20±6% decrease in basal metabolic rate (p<0.01, n=3) and a 22±9% decrease in maximal mitochondrial capacity (p<0.05, n=3) following CCCP injection. Rotenone induced 85±5% decrease in oxidative phosphorylation (p<0.001, n=3) and 83%, 87% in basal and maximal capacity as well. Glutamine oxidation rate (glutaminolysis) is juxtaposed to the right showing not significant 26±30% increase following troglitazone treatment, but a 382±115% increase due to rotenone exposure (p<0.03, n=3).

FIG. 7E shows that extracellular acidification rate (ECAR), a surrogate measure of lactate production, increased by 43±43% in troglitazone-treated cells, and decreased 21±12% in rotenone-treated cells (p>0.17, n=3). ^{∗} p<0.05, ^{∗∗} p<0.001 by student's T-test.

To confirm the findings static cultures of HepG2/C3A cells to 50 µM of rotenone and troglitazone and stained for mitochondrial membrane potential (MMP) at regular intervals (FIG. 7A). Cells showed a rapid 40±6% increase in MMP within 3 hours of exposure to rotenone (p<0.001, n=3) but signal was rapidly lost due to cell death within 6 hours. In contrast, exposure to troglitazone showed a steady 33±7% increase in MMP over 6 hours of exposure (p<0.001, n=3; FIG. 7B). To demonstrate that altered MMP leads to the observed changes in metabolic pathways the Seahorse Flux Analyzer was used to measure changes in oxidative phosphorylation, glutaminolysis and glycolysis on cells exposed to 50 µM of rotenone and troglitazone for 6 hours (FIGs. 7C-E). Rotenone induced 83±5% decrease in mitochondrial basal metabolic rate (p<0.0002, n=3) and 85±8% decrease in oxidative phosphorylation (p<0.0005, n=3). Glutaminolysis increased 4±1 folds (p<0.03, n=3; FIG. 7D), while glycolytic capacity was relatively unchanged (FIG. 7E). Changes induced by troglitazone were milder, resulting in a 20±6% decrease in mitochondrial basal metabolic rate (p<0.01, n=3) but no significant changes in glutaminolysis or glycolysis could be measured under static conditions although both fluxes showed a mild up-regulation (FIGs. 7D-E).

Table 1, below, provides calculated metabolic fluxes in nmol/min/10⁶ cells for untreated cells (normal), troglitazonetreated cells (mitochondrial stress) and rotenone-treated cells (mitochondrial dysfunction). Total glucose uptake rate was 2.4 nmol/min/10⁶ for untreated cells, 2.5 nmol/min/10⁶ for troglitazonetreated cells, and 1.0 nmol/min/10⁶ for rotenone-treated cells.

**Table 1**

| | Glycolysis | Respiration | Glutaminolysis | Lipogenesis |
|---|---|---|---|---|
| Normal | 1.49 | 0.31 | 0 | 0.62 |
| Mitochondrial Stress | 2.25 | 0.25 | 0.02 | 0 |
| Mitochondrial Dysfunction | 0.97 | 0.03 | 0.56 | 0 |

### Materials and Methods

### Bioreactor Fabrication

Bioreactors were fabricated from polymethyl methacrylate (PMMA) using CNC machining. Each unit was composed of two 50.8 mm circular support structures that fit standard 2" inserts, containing glass windows for microscopy. The bioreactor housed a removable polydimethylsiloxane (PDMS) microwell insert in which cells are protected from the negative effect of shear. Sealing around the microwells was realized with a rubber gasket creating an internal volume of 40 µl. Removable Microwell Insert PDMS microwell inserts were fabricated using laser cutting. Briefly, a thin sheet of PDMS (Dow Corning, USA) was cast to 0.7 mm height using a motorized film applicator (Erichsen, Germany) and cured at 70°C for 1 hour. Microwells were cut to 1.5 mm diameter, and a center-to-center distance of 3 mm using 355 nm, pulsed Nd-YAG laser (3D-Micromac, Germany). PDMS inserts were washed with 70% EtOH, nitrogen dried, and covalently bound to clean 0.5 mm thick glass coverslips (Schott, Elmsford, NY) using oxygen plasma activation.

### Cell Culture

HepG2/C3A cells were cultured under standard conditions in a humidified incubator at 37°C, under 5% CO2. Cells were obtained from the American Type Culture Collection (ATCC, USA). Cells were cultured in low glucose Modified Eagle Medium supplemented with 10% fetal calf serum (FCS), 100 U/ml penicillin and 100 µg/ml streptomycin (Sigma-Adlrich, USA). Medium was supplemented with 1% DMSO following bioreactor seeding to induce growth arrest and differentiation (16, 17).

### Bioreactor Seeding

PDMS microwell inserts were sterilized with 70% EtOH and 30 min exposure to UV light prior to cell seeding. Cells were trypsinized, counted and centrifuged at 300×g for 5 min at 4°C. The pellet was then mixed with 400 µg CPOx-50-RuP oxygen-sensing beads (Colibri Photonics, Germany) and resuspended in 100 µl of ice-cold solution of collagen type I (BD Biosciences, USA) for a final seeding density of 4×10⁶ cells/ml. The PDMS microwell insert was placed on ice and coated with ice-cold collagen solution for 5 min to remove air bubbles. Then 100 µl of collagen type I suspension containing cells and oxygen-sensing beads was layered on top the wells and incubated for 10 min on ice. The low temperature prevented premature collagen polymerization and the induction of cellular stress response pathways. Afterwards, excess cell and microbead suspension was gently wiped off the microwell insert using a sterile glass coverslip leading to a concentration of 100,000 cells and 20 oxygen-sensing beads per well. Variance in cell density between individual wells and between bioreactors was less than 10% measured by DNA content. The inoculated microwell insert was then incubated for 5 min at 37°C to polymerize the collagen. Following polymerization, the insert was immersed in 5 ml of cell culture medium and incubated for 20 min at 37°C prior to being sealed in the bioreactor housing. Bioreactors were then placed in a climate control chamber (Evotec, Germany) on an IX81 fluorescence microscope (Olympus, Japan). Bioreactors were continuously perfused with cell culture medium noted above supplemented with 10 mM HEPES and 1% DMSO at a flow rate of 2 µl/min. The automated and motorized microscope stage was equipped with a holder for three microreactors, allowing three experiments to run simultaneously.

### Quantitative Real Time Polymerase Chain Reaction (qRT-PCR)

RNA was isolated and purified utilizing Macherey-Nagel NucleoSpin RNA II kit according to manufacturer instructions. RNA concentration and purity was determined using NanoDrop ND-1000 spectrophotometer (Thermo Fisher Scientific, USA). cDNA was synthesized from 1 µg RNA sample using qScript cDNA SuperMix (Quanta BioSciences, USA) according to the manufacturer's protocol. Gene expression analysis was carried out utilizing KAPA SYBR FAST Universal 2X qPCR Master Mix (Kapa Biosystems, Boston, USA) on BioRad CFX96 Real-Time System (New South Wales, Australia), according to manufacturer's directions. Gene transcription was evaluated using the ΔΔCₜ method normalized to 60S ribosomal protein L32 (RPL32) or ubiquitin C (UBC).

### Finite Element Analysis

A computational fluid dynamic model was used to model pressure distribution, oxygen supply, and glucose consumption rates within the microwell bioreactor. A three-dimensional model of the bioreactor geometry was designed and meshed using an extremely fine mesh of 5 µm tetrahedral elements. Finite element simulations were carried out using COMSOL Multiphysics 4.3b, coupling the Navier-Stokes equations with the convection and diffusion model for transport of oxygen and glucose. Inlet oxygen concentration was atmospheric 0.2 mM with a diffusion coefficient of 1.8×10⁻⁹ m²/sec. Oxygen uptake rate was measured to be 1.8×10⁻⁹ mol/min/10⁶ cells. Inlet glucose concentration was 5.5 mM with a diffusion coefficient of 6×10⁻¹⁰ m²/sec, and uptake rate was measured under steady state condition to be 2.4×10⁻⁹ mol/min/10⁶ cells. Inlet lactate concentration was measured to be 1.5 mM, and production rate was measured to be 3×10⁻⁹ mol/min/10⁶ cells under steady state conditions.

### Chemical Compounds

Troglitazone (T2573), and rotenone (R8875) were purchased from Sigma-Aldrich (Schnelldorf, Germany) and diluted from H₂O or DMSO stock concentration 1:1000 into HepG2/C3A culture medium. Exposure onset was calculated to within ±1 min based on volumetric flow rate, considering the length of the tubing and bioreactor volume.

### Real-Time Oxygen Measurement

Real-time oxygen measurements were performed optically using lifetime-based luminescencequenching as previously described (11). Briefly, 50 µm diameter polystyrene microbeads were loaded with ruthenium-phenanthroline-based phosphorescence dye (CPOx-50-RuP) that shows a decrease in phosphorescence decay time as a function of oxygen concentration (FIG. 2C). Excited by a sinusoidal amplitude-modulated 532 nm LED source, the microbeads emit a sinusoidal amplitudemodulated light at 605 nm that is shifted in phase due to oxygen quenching (FIG. 2D). To overcome the superposition of in-phase background fluorescence that alters the phase of the detected signal, a 53.5 kHz and 31.3 kHz two-frequency phase modulation was used. This allowed screening out background interference (26-28). Measurements were carried out by averaging 5 consecutive 3 second exposures, followed by 17 seconds intervals, for a total of 100 seconds. Measurements were taken every 15 minutes for as long as 28 days with no phototoxicity, signal drift, or relevant loss of signal intensity. The apparatus consists of the OPAL package (Colibri Photonics, Germany) that is comprised of a control module, 532 nm LED, and a photomultiplier (PMT) detector mounted on the ocular of an IX83 Olympus microscope (Olympus, Japan). A filter cube with 531/40 (Ex), 607/70 (Em) was inserted in the optical light path during measurements.

### Evaluation of Toxicological End Points

PMDS microwell inserts were removed from the bioreactor following 24 hours exposure to rotenone or troglitazone. Cholestasis was evaluated using 5(6)-carboxy-2',7'-dichlorofluorescein diacetate (CDFDA) cell permeable dye which is metabolized to fluorescent CDF and secreted to active bile canaliculi by active multidrug resistance-associated protein 2 (MRP2). Briefly, cells were incubated with 2 µg/mL of CDFDA and 1 µg/mL of Hoechst 33342 for 20 min, and washed with PBS. Number of bile canaliculi foci was normalized for number of Hoechst-labeled nuclei. Apoptosis was evaluated using DeadEnd^{™} fluorometric TUNEL system (Promega, USA) according to manufacturer directions. Briefly, cells were fixed in 4% paraformaldehyde, permeabilized and exposed to fluorescein-12-dUTP and terminal deoxynucleotidyl transferase (TdT), dyeing apoptotic nuclei green. The reaction was subsequently stopped and the cells counterstained for Hoechst. A percent apoptotic nucleus was calculated by dividing the number of TUNEL to Hoechst positive nuclei.

### Measurement of Mitochondrial Stress in Isolated Mitochondria

Mitochondria were isolated using a Mitochondria Isolation Kit (Abcam, UK) per manufacturer instructions. Briefly, 10⁷ cells were collected and ruptured using 30 strokes in a pre-cooled dounce homogenizer. Homogenate was centrifuged twice at 1,000×g for 10 minutes at 4°C to remove debris, and finally at 12,000×g for 15 minutes at 4°C. Pellet containing the isolated mitochondria was resuspended in a protease inhibitor cocktail. Protein concentration was determined using Bradford. 20 µg of mitochondrial protein per well was plated in gelatin-coated SeaHorse XFp cell culture miniplate and centrifuged at 3,000×g for 20 minutes at 4°C in mitochondrial assay solution (MAS) as previously described (29). Oxygen consumption rate (OCR) was measured by the XFp Extracellular Flux Analyzer (Seahorse Biosciences, USA). Mitochondrial activity was profiled by successive injections of ADP (Sigma-Aldrich, Israel) and 50 µM of troglitazone, rotenone or DMSO control.

### Microfluidic Switchboard Fabrication

A computer-controlled microfluidic switchboard carried out automated sampling, calibration, and washing of the amperometric sensor unit, fabricated using two layer soft lithography. Briefly, an adherent layer of hexamethyldisilazane (Sigma-Aldrich) was spin coated at 4000 rpm for 30 seconds. Four layers of AZ-4652 (MicroChem) were sequentially spin coated at 800 rpm for 40 seconds, baked for 5 minutes at 100°C and exposed to UV light for 90 seconds. Curved AZ-4652 flow channels were molded by reflow using a gradual temperature increase of 35°C to 150°C over 3 hours followed by hard-baked overnight at 150°C. AZ-4652 channels dimensions were measured to be 100×25 µm (W×H). Control layer was fabricated separately using SU-8 photoresist (MicroChem), spin coated to 100 µm height, pre-baked for 25 minutes at 100°C, exposed to UV light for 15 seconds and postbaked for 12 minutes. Top layer SU-8 control channels dimensions were 100×100 µm (W×H). Master molds were treated with trichlorosilane (Sigma-Aldrich) for easier removal of polydimethylsiloxane (PDMS) after curing. Each layer was cast separately by replica molding, aligned and bonded via curing agent diffusion as previously described(30). The microfluidic switchboard was finally bonded to glass using oxygen plasma activation. The final device consists of 11 inlets and a common outlet that are regulated by self-addressable micromechanical valves.

### Flow System and Microcontroller

Dedicated microfluidic controller and flow circuit was previously described in detail [13]. Briefly, digitally controlled solenoid valves (Festo, Israel) were assembled into two pressure manifolds with a linear range of 0.15 to 6 bars. One manifold was connected to fluid reservoirs (Fluigent, Paris, France) perfusing the bioreactor, washing solution, atmospheric air, and calibration buffers. The second manifold was used to actuate the switchboard's control channels with a pressure of 1.05 bar. The switchboard flow outlet was connected to the PMMA housing of the amperometric sensors unit.

### Real-Time Glucose and Lactate Measurements

Amperometric glucose and lactate sensors were purchased from BioSensor Technology (Berlin, Germany) and embedded in a CNC-fabricated PMMA flow-chamber with an inner volume of 26 µl. The sensors are based on the enzymatic reactions of glucose oxidase, with a linear range of 0.5 mM to 30 mM, and lactate oxidase, with a linear range of 0.5 mM to 20 mM. Both sensors produce H₂O₂ in amounts proportional to the measured metabolite, which is detected with platinum electrodes under polarized condition. The microfluidic controller (13) was programmed to open a microfluidic valve every hour, allowing a 40 µl medium sample to flow through the switchboard and into the sensors unit. Amperometric measurement lasted 200 seconds and was followed by automated perfusion of PBS washing solution, CAL 4+M calibration buffer (Eschweiler, Germany), and 4 seconds of air bubble acting as a diffusion barrier between samples. Reading was carried out on EmStat3 OEM embedded potentiostat (PalmSens, Netherlands) connected to the central computer.

### Assessment of Cell Viability

Unless otherwise noted, cell viability was determined utilizing LIVE/DEAD Cytotoxicity kit (Molecular Probes, Eugene, OR) according to manufacturer's instructions. In brief, cultures were incubated with 2 µM Calcein AM and 3 µM ethidium homodimer-1 for 25 minutes. Hydrolysis by functional intracellular esterases causes live cells to fluoresce green, while the punctured membranes of dead cells permit ethidium homodimer-1 to bind DNA and fluoresce red. Cellular viability was calculated by live to dead ratio and normalized to negative control.

### Quantification of ATP/ADP ratio

Intracellular ATP and ADP quantification was carried out using Abcam ADP/ATP Assay Kit (#ab65313), according to manufacturer's instructions. Briefly, reaction buffer was pre-incubated at room temperature for 2 hours prior to beginning of measurements, to burn off low-level ATP contamination, as suggested by the manufacturer. Cells were incubated in nucleotide releasing buffer for 5 min at room temperature, with gentle shaking. Background bioluminescence reading of the reaction mix was taken for calibration, followed by 2 min incubation of 50 µl sample solution in each reaction well, and a bioluminescence reading to measure cellular ATP content. ADP converting enzyme was then added, as instructed, following incubation of 2 min at room temperature to measure total ADP+ATP.

### Measurement of Oxidative Phosphorylation and Mitochondrial Stress

HepG2/C3A cells were plated in gelatin-coated SeaHorse XFp cell culture miniplates (Seahorse Bioscience, USA) and cultured for 24 hours. Subsequently, cultures were treated with medium containing 50 µM troglitazone for 6 hours, 50 µM rotenone for 3 hours, or vehicle control prior to initiation of assay. Mitochondrial Stress Test assay was conducted per manufacturer instructions (29). Briefly, cells were incubated in unbuffered XF Base Medium supplemented with 2 mM glutamine, 1 mM sodium pyruvate, and 10 mM glucose (pH 7.4) for 1 hour at 37°C in a non-CO2 incubator. Basal oxygen consumption rate (OCR) was measured for 30 min, followed by injection of 1 µM oligomycin, a mitochondrial Complex IV inhibitor that completely blocks oxidative phosphorylation. The decrease in OCR due to oligomycin treatment is defined as oxidative phosphorylation rate. 1 µM carbonyl cyanide-4 (trifluoromethoxy) phenylhydrazone (FCCP), an uncoupling agent, is added at 60 min to measure maximal mitochondrial activity followed by complete inhibition at 90 min using a mixture of 0.5 µM antimycin A and rotenone, mitochondrial Complex III and Complex I inhibitors. Basal extracellular acidification rates (ECAR) was measured for 30 min, in the presence of 10 mM glucose, followed by injection of 1 µM oligomycin. The increase in ECAR due to oligomycin treatment was defined as glycolytic capacity. Data are presented normalized to 106 cells as determined by Hoechst DNA content assay.

### Measurement of Glutamine Oxidation

HepG2/C3A cells were plated in gelatin-coated XFp cell culture miniplates and cultured for 24 hours. Subsequently, cultures were treated with medium containing 50 µM troglitazone for 6 hours, 50 µM rotenone for 3 hours, or vehicle control prior to initiation of assay. Glutamine oxidation assay was conducted per manufacturer instructions. Briefly, cells were incubated in unbuffered XF Base Medium supplemented with 2 mM Glutamine, 1 mM sodium pyruvate, and 10 mM glucose (pH 7.4) for 1 hour at 37° C in a non-CO2 incubator. Glutamine oxidation was profiled by successive injections of 3 µM BPTES, an allosteric inhibitor of glutaminase (GLS1), and a mixture of 4 µM etomoxir, a carnitine palmitoyl-transferase 1A (CPT1A) inhibitor and 2 µM UK5099 a mitochondrial pyruvate carrier inhibitor, blocking fatty acid oxidation and oxidative phosphorylation, respectively. The decrease in baseline OCR due to BPTES treatment was defined as the rate of glutaminolysis. Data are presented normalized to 10⁶ cells as determined by Hoechst DNA content assay.

### Evaluating Mitochondrial Membrane Potential

JC-1 cell permeable dye was purchased from Life Technologies (New York, NY). HepG2/C3A cells were incubated with 50 µM of troglitazone or rotenone for varying durations. To evaluate mitochondrial membrane potential at each time point, cells were incubated with 5µM JC-1 dye for 30 min, washed with PBS and imaged by confocal microscopy on a LSM700 (Zeiss, Germany). The JC-1 dye exhibits potential-dependent accumulation in mitochondria, indicated by a fluorescence emission shift from 529 to 590 nm due to the formation of J-aggregates. Consequently, mitochondrial depolarization is quantified by dividing red over green fluorescence intensity.

### Metabolic Pathways and ATP Production

FIG. 9A shows images of HepG2/C3A cells stained for PAS and hematoxylin, following 12 hours exposure to drugs.

FIG. 9B demonstrates that glycogen content is unchanged between the three conditions.

Glucose uptake, oxygen uptake and lactate production rates were measured by calculating the change in metabolite concentration between the bioreactor in- and outflow, as function of perfusion rate and cell number as described above. Metabolic rates were calculated assuming negligible contribution to oxygen uptake by fatty acid oxidation and enzymatic activity. Low level of lipids in the culture medium ensured that fatty acid uptake was 50-fold lower than glucose while glutamine contribution to the Krebs cycle was minor (results). Periodic acid-Schiff (PAS) staining showed no significant change in glycogen content following 12 hours exposure to the drugs (FIGs. 9A and 9B).

Based on these assumptions, oxidative phosphorylation flux was calculated by dividing oxygen uptake rate by six. 32 ATP molecules generated by complete oxidation of 1 molecule of glucose were estimated. Glycolysis flux was calculated by dividing lactate production rate by two, with maximal rate defined by glucose uptake rate minus the oxidative phosphorylation flux. ATP production in glycolysis was estimated to be 2 molecules per molecule of glucose. It was assumed that any glucose left over was directed toward lipogenesis, as the contribution of pentose phosphate pathway in nonproliferating cells is minor (31). Finally, it was assumed that excess lactate was produced by glutaminolysis, and confirmed the assumption using offline measurement of glutamine uptake (FIGs. 10A and 10B). ATP production in glutaminolysis was estimated to be 3 molecules per molecule of lactate generated.

### Statistical Analysis

Experiments were repeated 2 to 3 times with triplicate samples for each experimental condition. Data from representative experiments are presented, whereas similar trends were seen in multiple trials. A parametric two-tailed Student's t-test was used for calculating significant differences between groups. All n values represent the number of biological samples analyzed per condition. All error bars indicate ± standard deviation.

### Glycogen Quantification

Intracellular glycogen quantification was carried out using Periodic acid-Schiff (PAS) staining (Sigma-Aldrich). Briefly, HepG2/C3A cells were treated for 12 hours with 50 µM troglitazone, rotenone, or vehicle control and fixed for 15 min in 4% PFA. Cells were washed in distilled water, and incubated for 5 min, at room temperature with the periodic acid solution. Next the cells were incubated in Schiff's reagent for 15 min at room temperature, washed 3 times and counterstained in hematoxylin solution Gill No. 3 for 90 seconds at room temperature, and left to dry. Quantification was performed using Image J software and presented as the ratio between PAS and hematoxylin staining.

### Glutamine Uptake

Glutamine concentration was determined using glutamine and glutamate determination kit (Sigma-Aldrich). Bioreactors containing HepG2/C3A spheroids were set up as described in the main text and perfused at 2 µl/min. Perfusate samples were collected for 60 minutes for a total of 120 µl per sample. Glutamine was quantified according to manufacturer directions.

### Discussion

In this Example a robust microfluidic platform combining liver-on-chip technology with automated microfluidic analysis of glucose metabolism and mitochondrial function (FIGs. 2A-F) was established. The system utilizes 1% DMSO to growth-arrest self-assembled aggregates of HepG2/C3A cells maintained under physiological conditions (FIGs. 3A-G). Cultures stabilized within 4 days displaying strong elevation of CYP450 expression (FIGs. 3F-G) and MRP2-active apical surfaces (FIG. 4F). At steady state, 13±5% of the glucose consumed by growth-arrested HepG2/C3A cells in the bioreactor was directed towards oxidative phosphorylation with minor to negligible contributions from glutamine and lipid oxidation.

One advantage of the platform is the integration of medical-grade commercial sensors in a modular off-chip unit. These off-the-shelf components reduce fabrication costs and can be replaced as needed without pausing the experiment. This is particularly useful in electrochemical sensors whose activity is dependent on membrane integrity and enzymatic function. Off-chip measurement also allowed automating sensor calibration, and create sharp chemical gradients, insuring measurement stability for several days in culture. This stands in contrast to prior reports of integrated on-chip glucose and lactate sensors that offer less than 1-3 hours of operation before signal drift requires recalibration [23]. Stability of the sensors permitted over 24 hours of continuous sampling (FIGs. 6A-H). In fact, while oxygen uptake dropped within minutes after exposure to rotenone and troglitazone (FIG. 4H) the metabolic shift from oxidative phosphorylation to glycolysis occurred only after 3 and 6 hours of exposure, respectively (FIGs. 6C-F). Analysis of MMP showed a similar time frame and a gradual loss of function, coupled with a gradual change in glucose and lactate fluxes, suggesting that enzymatic rather than transcriptional changes are responsible for the metabolic shift.

A second advantage of the platform is the optical measurement of oxygen using tissue-embedded micro-sensors (FIGs. 2A-F). Optical sensors are advantageous over Clark-type electrodes as the latter consume oxygen during measurement and require frequent recalibration [12]. While standard optical sensors are affected by changes in optical focus, background or particle migration, the approach of the present embodiments focused on measurement of phase-shift that is independent of changes in signal amplitude (11). Processes such as cell migration and necrosis that change the number and location of microbeads during the experiment have no effect on the measurement permitting continuous measurements of oxygen uptake for over 28 days in vitro (FIG. 3F). In fact, the ability to measure oxygen uptake rates during stable stimulation stands in contrast to other approaches in the field that either circulate medium to increase signal intensity [24] or measure under static conditions (25) rather than at steady state.

The integration of both sensing modalities allowed identifying differences in the metabolic response to rotenone and troglitazone that appear to be related to the severity of mitochondrial damage. Exposure to the anti-diabetic drug troglitazone (Rezulin^{©}) induced the expected response as cells diverted glucose from metabolic pathways such as lipogenesis toward lactate, maintaining 97% of cellular ATP production and normal ATP/ADP ratio (FIG. 6G). This switch was not previously detected as the Warburg effect often dominates cell metabolism in culture and cell viability is unaffected. While drug effect at this concentration appears minor, it could lead to an idiosyncratic response in compromised patients.

In contrast to the troglitazone response, cellular adaptation to rotenone-induced mitochondrial dysfunction was surprising. Cells showed a 59% decrease in glucose uptake, suggesting an accumulation of intermediates led to a block in downstream glycolysis. The switch to compensatory mechanisms was gradual, as lactate over glucose ratio increased above 2, and glutamine uptake increased, demonstrating a shift toward glutaminolysis (FIGs. 7C-E).

### EXAMPLE 2

Following are representative types of assays that can be employed using the method and system of the present embodiments.

### Measurement of the Basal Respiration and Acidification Rates of C2C12 Myoblasts

C2C12 murine skeletal muscle cells can be seeded on membranes which can then be incubated at 37 °C, for a period of, *e.g.,* 12 hours.

Bicarbonate (NaHCO₃)-free DMEM Medium can be added, and oxygen, glucose and lactate can be monitored. The average rate of change of each analyte can be calculated.

To determine the extracellular flux rates of O₂ and CO₂, the rates of change of partial pressures can be divided by volume of each analytes to result in a value. The rate of acidification can be expressed in, for example, protons per unit time by calculating the number of available electrons in the media buffer within the known sample volume.

### Measurement of Basal Respiration and Acidification Rates for Various Cell Densities

The relationship between cell number and oxygen and CO₂ flux rates can be investigated. Varying numbers of C2C12 myoblasts can be seeded and incubated as further detailed hereinabove. Bicarbonate (NaHCO₃)-free DMEM Medium can be added, and oxygen, glucose and lactate can be monitored. The average rate of change of each analyte can be calculated.

Increased analyte flux rates in a near-linear fashion can indicate an increasing cell density.

### The Effect of 2,4 DNP on C2C12 Myoblasts

The chemical compound 2,4 DNP can be used to uncouple mitochondrial respiration from ATP synthesis by disassociating the linkage between the respiratory chain and the phosphorylation system. In the presence of this compound, oxygen consumption will increase dramatically, while proton flux remains relatively constant.

C2C12 myoblasts can be seeded and incubated as further detailed hereinabove. Bicarbonate (NaHCO₃)-free DMEM Medium can be added, and oxygen, glucose and lactate can be monitored to determine a control baseline for each analyte flux. Once the baseline is established, a sequence of experiments can be performed where varying doses of 2,4 DNP are added to the cell media and a measurement of analyte flux rates is performed. A control experiment using the highest dose of 2,4 DNP, but without cells can also be performed.

### The Effect of Rotenone on C2C12 Myoblasts

Rotenone inhibits cellular respiration by blocking NADH dehydrogenase in the respiratory chain. C2C12 Myoblasts can be used to show this effect.

C2C12 myoblasts can be seeded and incubated as further detailed hereinabove. Bicarbonate (NaHCO₃)-free DMEM Medium can be added, and oxygen, glucose and lactate can be monitored to determine a control baseline for each analyte flux.

Once the baseline is established, a sequence of experiments can be performed where varying doses of Rotenone is added to the cell media and a measurement of analyte flux is performed. A control experiment using the highest dose of Rotenone, but without cells can also be performed.

### Measurement of Respiration and Acidification Rate Changes Resulting from Cell Proliferation

C2C12 myoblasts can be seeded and incubated as further detailed hereinabove. Bicarbonate (NaHCO₃)-free DMEM Medium can be added to inhibit proliferation. About 12 hours after being seeded, the cells can be placed in DMEM serum-free media. After about 24 hours, part half of the cells can be switched to DMEM serum-containing media to stimulate proliferation, while the other part can be maintained in serum-free media. Oxygen, glucose and lactate can be monitored in the presence of NaHCO₃-free DMEM Medium.

### Measurement of G-Protein Coupled Receptor Activation in CHO-M3 Cells

Stimulation of transmembrane receptors often causes a rapid increase in extracellular acidification rate, resulting primarily from acute activation of ion exchange pumps. In this experiment, the technique of the present embodiments can be used to detect a change in extracellular acidification rate following treatment of cells with a receptor agonist.

Chinese hamster ovary (CHO) cells can be transfected to over-express the muscarinic receptor subtype m3. The cells can be placed on the plate of the present embodiments to monitor oxygen, glucose and lactate as further detailed hereinabove, following treatment with, for example, the general acetylcholine receptor agonist, Carbachol.

### REFERENCES

[1] Huh D, et al. (2010) Reconstituting Organ-Level Lung Functions on a Chip. Science 328(5986):1662-1668.
[2] Monga SP, et al. (2005) Mouse fetal liver cells in artificial capillary beds in three-dimensional four-compartment bioreactors. Am J Pathol 167(5):1279-1292.
[3] Allen JW & Bhatia SN (2003) Formation of steady-state oxygen gradients in vitro: application to liver zonation. Biotechnol Bioeng 82(3):253-262.
[4] Khademhosseini A, et al. (2005) Cell docking inside microwells within reversibly sealed microfluidic channels for fabricating multiphenotype cell arrays. Lab Chip 5(12):1380-1386.
[5] Sivaraman A, et al. (2005) A microscale in vitro physiological model of the liver: predictive screens for drug metabolism and enzyme induction. Curr Drug Metab 6(6):569-591.
[6] Adeleye Y, et al. (2014) Implementing Toxicity Testing in the 21st Century (TT21C): Making safety decisions using toxicity pathways, and progress in a prototype risk assessment. Toxicology (1879-3185).
[7] Vinken M (2013) The adverse outcome pathway concept: a pragmatic tool in toxicology. Toxicology 312(1879-3185 (Electronic)): 158-165.
[8] Nelms MD, et al. (2015) Proposal of an in silico profiler for categorisation of repeat dose toxicity data of hair dyes. Arch Toxicol 89(5):733-741.
[9] Moussaieff A, et al. (2015) Glycolysis-Mediated Changes in Acetyl-CoA and Histone Acetylation Control the Early Differentiation of Embryonic Stem Cells. Cell Metabolism 21(3):392-402.
[10] Vinken M (2015) Adverse Outcome Pathways and Drug-Induced Liver Injury Testing. Chemical Research in Toxicology 28(7):1391-1397.
[11] Prill S, et al. (2015) Real-time monitoring of oxygen uptake in hepatic bioreactor shows CYP450-independent mitochondrial toxicity of acetaminophen and amiodarone. Arch Toxicol: 1-11.
[12] Alborzinia H, et al. (2011) Real-Time Monitoring of Cisplatin-Induced Cell Death. PLoS ONE 6(5):e19714.
[13] Ezra E, et al. (2015) Microprocessor-based integration of microfluidic control for the implementation of automated sensor monitoring and multithreaded optimization algorithms. Biomed Microdevices 17(4):1-9.
[14] Rowlands JC, Sander M, Bus JS, & FutureTox Organizing C (2014) FutureTox: Building the Road for 21st Century Toxicology and Risk Assessment Practices. Toxicol. Sci. 137(2):269- 277.
[15] Tilles AW, Baskaran H, Roy P, Yarmush ML, & Toner M (2001) Effects of oxygenation and flow on the viability and function of rat hepatocytes cocultured in a microchannel flat-plate bioreactor. Biotechnol Bioeng 73(5):379-389.
[16] Sainz B & Chisari FV (2006) Production of Infectious Hepatitis C Virus by Well-Differentiated, Growth-Arrested Human Hepatoma-Derived Cells. Journal of Virology 80(20):10253-10257.
[17] Fiore M, Zanier R, & Degrassi F (2002) Reversible G1 arrest by dimethyl sulfoxide as a new method to synchronize Chinese hamster cells. Mutagenesis 17(5):419-424.
[18] Cukierman E, Pankov R, Stevens DR, & Yamada KM (2001) Taking cell-matrix adhesions to the third dimension. Science 294(5547):1708-1712.
[19] Baharvand H, Hashemi SM, Ashtian SK, & Farrokhi A (2006) Differentiation of human embryonic stem cells into hepatocytes in 2D and 3D culture systems in vitro. International Journal of Developmental Biology 50(7):645-652.
[20] Siddiqui MA, et al. (2013) Rotenone-induced oxidative stress and apoptosis in human liver HepG2 cells. Mol Cell Biochem 384(1-2):59-69.
[21] Julie NL, Julie IM, Kende AI, & Wilson GL (2008) Mitochondrial dysfunction and delayed hepatotoxicity: another lesson from troglitazone. Diabetologia 51(11):2108-2116.
[22] Masubuchi Y (2006) Metabolic and Non-Metabolic Factors Determining Troglitazone Hepatotoxicity: A Review. Drug Metabolism and Pharmacokinetics 21(5):347-356.
[23] Eklund S, et al. (2009) Metabolic Discrimination of Select List Agents by Monitoring Cellular Responses in a Multianalyte Microphysiometer. Sensors 9(3):2117.
[24] Domansky K, et al. (2010) Perfused multiwell plate for 3D liver tissue engineering. Lab on a Chip 10(1):51-58.
[25] Beeson CC, Beeson GC, & Schnellmann RG (2010) A high-throughput respirometric assay for mitochondrial biogenesis and toxicity. Analytical Biochemistry 404(1):75-81.
[26] Ast C, Schmalzlin E, Lohmannsroben HG, & van Dongen JT (2012) Optical Oxygen Microand Nanosensors for Plant Applications. Sensors 12(6):7015-7032.
[27] Schmalzlin E, et al. (2005) An optical multifrequency phase-modulation method using microbeads for measuring intracellular oxygen concentrations in plants. Biophysical Journal 89(2):1339-1345.
[28] Lohmannsroben HG, Beck M, Hildebrandt N, Schmalzlin E, & van Dongen JT (2006) New challenges in biophotonics: Laser-based fluoroimmuno analysis and in-vivo optical oxygen monitoring - art. no. 61570E. Workshop on Laser Applications in Europe, Proceedings of the Society of Photo-Optical Instrumentation Engineers (Spie), eds Gries W & Pearsall TP (Spie- Int Soc Optical Engineering, Bellingham), Vol 6157, pp E1570-E1570.
[29] Zhang J, et al. (2012) Measuring energy metabolism in cultured cells, including human pluripotent stem cells and differentiated cells. Nat. Protocols 7(6):1068-1085.
[30] Melin J & Quake SR (2007) Microfluidic large-scale integration: the evolution of design rules for biological automation. Annual review of biophysics and biomolecular structure 36:213-231.
[31] Ouattara DA, et al. (2012) Metabolomics-on-a-chip and metabolic flux analysis for label-free modeling of the internal metabolism of HepG2/C3A cells. Molecular BioSystems 8(7):1908- 1920.

## Claims

1. A method for biosensing of cells, comprising
(a) subjecting the cells to a continuous single-pass perfusion wherein said perfusion comprises a perfusion medium which comprises a proliferation inhibitor, wherein said cells comprise microparticles or nanoparticles embedded therebetween, wherein said microparticles or nanoparticles serve as oxygen sensors,
(b) measuring glucose and lactate directly using electrochemical sensors, wherein electrical signal from said electrochemical sensors and optical signals from said oxygen sensors are collected over the same time period, and
(c) analyzing signals of said oxygen sensors and of said electrochemical sensors to thereby determine one or more physiological parameters that are characteristic to the cells.

2. The method of claim 1, wherein said one or more physiological parameters is glycolytic flux of the cells.

3. The method of claim 2, wherein said one or more physiological parameters indicative of said glycolytic flux is glycolytic capacity.

4. The method of claim 3, wherein said glycolytic capacity is determined following perfusion with a saturating concentration of glucose.

5. The method of claim 2, wherein said glycolytic flux is glycolytic reserve.

6. The method of claim 5, wherein said glycolytic reserve is determined following perfusion with a glycolysis inhibitor.

7. The method of claim 6, wherein said glycolysis inhibitor is 2-deoxyglucose, which inhibits glycolysis by binding to hexokinase.

8. The method of claim 2, wherein said glycolytic flux is non-glycolytic acidification.

9. The method of claim 1, wherein said one or more physiological parameters is mitochondrial function.

10. The method of claim 9, wherein said mitochondrial function is basal respiration.

11. The method of claim 9, wherein said mitochondrial function is ATP-linked respiration, H⁺ leak, maximal respiration or spare respiratory capacity.

12. The method of claim 1, wherein said one or more physiological parameters is non-mitochondrial respiration.

13. The method of claim 12, wherein said non-mitochondrial respiration is determined following perfusion of rotenone and antimycin A.

14. The method of claim 1, wherein said perfusion medium comprises a glycolysis inhibitor, a mitochondrial pyruvate carrier (MPC) inhibitor, a glutaminase GLS1 inhibitor and/or a Carnitine Palmitoyltransferase 1A (CPT1A) inhibitor.

15. The method of claim 1, wherein said perfusion medium comprises glucose.

16. The method of claim 1, wherein said perfusion medium comprises at least one of a drug, a drug candidate, a toxin and a nutrient and wherein the method comprising assessing change of oxygen uptake, change of glucose uptake and change of lactate production in the presence of the at least one of a drug, a drug candidate, a toxin and a nutrient.

## Patentansprüche

1. Verfahren zum Biosensing von Zellen, umfassend
(a) Unterziehen der Zellen einer kontinuierlichen Single-Pass-Perfusion, wobei die Perfusion ein Perfusionsmedium umfasst, das einen Proliferationsinhibitor umfasst, wobei die Zellen Mikropartikel oder Nanopartikel umfassen, die dazwischen eingebettet sind, wobei die Mikropartikel oder Nanopartikel als Sauerstoffsensoren dienen,
(b) direktes Messen von Glukose und Laktat unter Verwendung elektrochemischer Sensoren, wobei elektrische Signale von den elektrochemischen Sensoren und optische Signale von den Sauerstoffsensoren über denselben Zeitraum gesammelt werden, und
(c) Analysieren der Signale der Sauerstoffsensoren und der elektrochemischen Sensoren, um dadurch einen oder mehrere physiologische Parameter zu bestimmen, die für die Zellen charakteristisch sind.

2. Verfahren nach Anspruch 1, wobei der eine oder die mehreren physiologischen Parameter der glykolytische Fluss der Zellen ist.

3. Verfahren nach Anspruch 2, wobei der eine oder die mehreren physiologischen Parameter, die den glykolytischen Fluss anzeigen, die glykolytische Kapazität ist.

4. Verfahren nach Anspruch 3, wobei die glykolytische Kapazität nach Perfusion mit einer sättigenden Glukosekonzentration bestimmt wird.

5. Verfahren nach Anspruch 2, wobei der glykolytische Fluss die glykolytische Reserve ist.

6. Verfahren nach Anspruch 5, wobei die glykolytische Reserve nach Perfusion mit einem Glykolyse-Inhibitor bestimmt wird.

7. Verfahren nach Anspruch 6, wobei der Glykolyse-Inhibitor 2-Desoxyglucose ist, die die Glykolyse durch Bindung an Hexokinase hemmt.

8. Verfahren nach Anspruch 2, wobei der glykolytische Fluss eine nichtglykolytische Ansäuerung ist.

9. Verfahren nach Anspruch 1, wobei der eine oder die mehreren physiologischen Parameter die mitochondriale Funktion ist.

10. Verfahren nach Anspruch 9, wobei die mitochondriale Funktion die Basalatmung ist.

11. Verfahren nach Anspruch 9, wobei die mitochondriale Funktion die ATP-gebundene Atmung, das H⁺-Leck, die maximale Atmung oder die freie Atmungskapazität ist.

12. Verfahren nach Anspruch 1, wobei der eine oder die mehreren physiologischen Parameter die nicht-mitochondriale Atmung ist.

13. Verfahren nach Anspruch 12, wobei die nicht-mitochondriale Atmung nach Perfusion von Rotenon und Antimycin A bestimmt wird.

14. Verfahren nach Anspruch 1, wobei das Perfusionsmedium einen Glykolyse-Inhibitor, einen mitochondrialen Pyruvat-Träger (MPC)-Inhibitor, einen Glutaminase-GLS1-Inhibitor und/oder einen Carnitin-Palmitoyltransferase 1A (CPT1A)-Inhibitor umfasst.

15. Verfahren nach Anspruch 1, wobei das Perfusionsmedium Glukose umfasst.

16. Verfahren nach Anspruch 1, wobei das Perfusionsmedium mindestens eines von einem Arzneimittel, einem Arzneimittelkandidaten, einem Toxin und einem Nährstoff umfasst und wobei das Verfahren die Bewertung der Änderung der Sauerstoffaufnahme, der Änderung der Glukoseaufnahme und der Änderung der Laktatproduktion in Gegenwart des mindestens einen von einem Arzneimittel, einem Arzneimittelkandidaten, einem Toxin und einem Nährstoff umfasst.

## Revendications

1. Procédé de biodétection de cellules, comprenant
(a) la soumission des cellules à une perfusion continue en un seul passage dans lequel ladite perfusion comprend un milieu de perfusion qui comprend un inhibiteur de la prolifération, dans lequel lesdites cellules comprennent des microparticules ou des nanoparticules intégrées entre celles-ci, dans lequel lesdites microparticules ou nanoparticules servent de détecteurs d'oxygène,
(b) la mesure du glucose et du lactate directement à l'aide de capteurs électrochimiques, dans lequel le signal électrique desdits capteurs électrochimiques et les signaux optiques desdits capteurs d'oxygène sont collectés sur la même période de temps, et
(c) l'analyse des signaux desdits capteurs d'oxygène et desdits capteurs électrochimiques pour ainsi déterminer un ou plusieurs paramètres physiologiques qui sont caractéristiques des cellules.

2. Procédé selon la revendication 1, dans lequel lesdits un ou plusieurs paramètres physiologiques sont un flux glycolytique des cellules.

3. Procédé selon la revendication 2, dans lequel lesdits un ou plusieurs paramètres physiologiques indiquant ledit flux glycolytique sont la capacité glycolytique.

4. Procédé selon la revendication 3, dans lequel ladite capacité glycolytique est déterminée après une perfusion avec une concentration saturante de glucose.

5. Procédé selon la revendication 2, dans lequel ledit flux glycolytique est la réserve glycolytique.

6. Procédé selon la revendication 5, dans lequel ladite réserve glycolytique est déterminée après une perfusion avec un inhibiteur de la glycolyse.

7. Procédé selon la revendication 6, dans lequel ledit inhibiteur de la glycolyse est un 2-désoxyglucose, qui inhibe la glycolyse par liaison à une hexokinase.

8. Procédé selon la revendication 2, dans lequel ledit flux glycolytique est une acidification non glycolytique.

9. Procédé selon la revendication 1, dans lequel lesdits un ou plusieurs paramètres physiologiques sont la fonction mitochondriale.

10. Procédé selon la revendication 9, dans lequel ladite fonction mitochondriale est la respiration basale.

11. Procédé selon la revendication 9, dans lequel ladite fonction mitochondriale est la respiration liée à l'ATP, une fuite de H⁺, une respiration maximale ou une capacité respiratoire de réserve.

12. Procédé selon la revendication 1, dans lequel lesdits un ou plusieurs paramètres physiologiques sont la respiration non mitochondriale.

13. Procédé selon la revendication 12, dans lequel ladite respiration non mitochondriale est déterminée après une perfusion de roténone et d'antimycine A.

14. Procédé selon la revendication 1, dans lequel ledit milieu de perfusion comprend un inhibiteur de glycolyse, un inhibiteur du transporteur mitochondrial du pyruvate (MPC), un inhibiteur de la glutaminase GLS1 et/ou un inhibiteur de la carnitine palmitoyltransférase 1A (CPT1A).

15. Procédé selon la revendication 1, dans lequel ledit milieu de perfusion comprend du glucose.

16. Procédé selon la revendication 1, dans lequel ledit milieu de perfusion comprend au moins un médicament, un candidate médicament, une toxine et un nutriment et dans lequel le procédé comprend l'évaluation d'une modification de l'absorption d'oxygène, d'une modification de l'absorption du glucose et d'une modification de la production du lactate en présence de l'au moins un d'un médicament, d'un candidat médicament, d'une toxine et d'un nutriment.
